# EUROPEAN PATENT SPECIFICATION

(11) **EP 2 683 742 B1**
(45) Date of publication and mention of the grant of the patent: **19.12.2018**
(21) Application number: 11861101.1
(22) Date of filing: 15.09.2011
(51) Int. Cl.: C07K 19/00, C12N 15/62, C12N 15/63, C12N 15/86, A61P 9/10, A61P 3/10

(54) **NPP1 FUSION PROTEINS**
NPP1-FUSIONSPROTEINE
PROTÉINES HYBRIDES NPP1

(30) Priority: 11.03.2011 WO PCT/US2011/028233
(43) Date of publication of application: 15.01.2014
(73) Proprietor: Alexion Pharmaceuticals, Inc., New Haven, CT 06510 (US)
(72) Inventor: QUINN, Anthony, Chestnut Hill, Massachusetts 02467 (US); HARVEY, Alex J., Athens, Georgia 30606 (US); XIA, Zhinan, Wellesley, Massachusetts 02481 (US)
(74) Representative: Mathys & Squire LLP
(86) International application number: PCT/US2011/051858
(87) International publication number: WO 2012/125182

(56) References cited:
- WO-A2-2011/113027
- DE-U1- 20 121 960
- LOMASHVILI KOBA A ET AL: "Phosphate-induced vascular calcification: Role of pyrophosphate and osteopontin", JOURNAL OF THE AMERICAN SOCIETY OF NEPHROLOGY, vol. 15, no. 6, June 2004 (2004-06), pages 1392-1401, XP002726211, ISSN: 1046-6673
- W CHARLES O'NEILL ET AL: "Treatment with pyrophosphate inhibits uremic vascular calcification", KIDNEY INTERNATIONAL, vol. 79, no. 5, 1 December 2010 (2010-12-01), pages 512-517, XP055042109, ISSN: 0085-2538, DOI: 10.1038/ki.2010.461
- RUTSCH FRANK ET AL: "Mutations in ENPP1 are associated with 'idiopathic' infantile arterial calcification.", NATURE GENETICS, vol. 34, no. 4, August 2003 (2003-08), pages 379-381, XP002726212, ISSN: 1061-4036
- MILLÁN JOSÉ LUIS ET AL: "Enzyme replacement therapy for murine hypophosphatasia", JOURNAL OF BONE AND MINERAL RESEARCH, BLACKWELL SCIENCE, INC, vol. 23, no. 6, 1 June 2008 (2008-06-01), pages 777-787, XP002585178, ISSN: 0884-0431, DOI: 10.1359/JBMR.071213 [retrieved on 2007-12-17]
- Kristen Johnson ET AL: "Linked Deficiencies in Extracellular PPi and Osteopontin Mediate Pathologic Calcification Associated With Defective PC-1 and ANK Expression", JOURNAL OF BONE AND MINERAL RESEARCH, 1 June 2003 (2003-06-01), pages 994-1004, XP055073713, Retrieved from the Internet: URL:http://onlinelibrary.wiley.com/store/1 0.1359/jbmr.2003.18.6.994/asset/5650180606 _ftp.pdf?v=1&t=hjskzgfr&s=50b03ad0b45518ac e4fe975087e35c9195f49fc5 [retrieved on 2013-07-31]
- RIK GIJSBERS ET AL.: 'Functional characterization of the non-catalytic ectodomains of the nucleotide pyrophosphatase/phosphodiesterase NPP1' BIOCHEM. J. vol. 371, 2003, pages 321 - 330, XP055116645
- AARON SCHETTER ET AL.: 'Nucleoporins NPP-1, NPP-3, NPP-4, NPP-11 and NPP-13 are required for proper spindle orientation in C. elegans' DEVELOPMENTAL BIOLOGY vol. 289, 02 December 2005, pages 360 - 371, XP024943696
- RIK GIJSBERS ET AL.: 'The hydrolysis of lysophospholipids and nucleotides by autotaxin (NPP2) involves a single catalytic site' FEBS LETTERS vol. 538, 2003, pages 60 - 64, XP004842825
- KRISTEN JOHNSON ET AL.: 'Chondrogenesis Mediated by PPi Depletion Promotes Spontaneous Aortic Calcification in NPP1-/- Mice' ARTERIOSCLER THROMB VASC BIOL. vol. 25, April 2005, pages 686 - 691, XP055118308
- CRISTIANA STEFAN ET AL.: 'NPP-type ectophosphodiesterases:unity in diversity' TREND IN BIOCHEMICAL SCIENCES vol. 30, no. 10, October 2005, pages 542 - 550, XP027637390
- LOMASHVILI K A ET AL: "Phosphate-induced vascular calcification: Role of pyrophosphate and osteopontin", JOURNAL OF THE AMERICAN SOCIETY OF NEPHROLOGY, WILLIAMS AND WILKINS, BALTIMORE, MD, US, vol. 15, no. 6, 1 June 2004 (2004-06-01), pages 1392-1401, XP002726211, ISSN: 1046-6673, DOI: 10.1097/01.ASN.0000128955.83129.9C
- HUANG ET AL: "Receptor-Fc fusion therapeutics, traps, and MIMETIBODY technology", CURRENT OPINION IN BIOTECHNOLOGY, LONDON, GB, vol. 20, no. 6, 1 December 2009 (2009-12-01), pages 692-699, XP026778880, ISSN: 0958-1669, DOI: 10.1016/J.COPBIO.2009.10.010 [retrieved on 2009-11-04]
- SCHMIDT STEFAN R: "Fusion-proteins as biopharmaceuticals--applications and challenges.", CURRENT OPINION IN DRUG DISCOVERY & DEVELOPMENT MAR 2009, vol. 12, no. 2, March 2009 (2009-03), pages 284-295, ISSN: 2040-3437

## Description

### BACKGROUND

Ectonucleotide pyrophosphatase/phosphodiesterase 1 (NPP1/ENPP1/PC-1) is a type II transmembrane glycoprotein that forms a homodimer. The protein cleaves a variety of substrates, including phosphodiester bonds of nucleotides and nucleotide sugars and pyrophosphate bonds of nucleotides and nucleotide sugars. NPP1 protein functions to hydrolyze nucleoside 5' triphosphtase to either corresponding monophosphates and also hydrolyzes diadenosine polyphosphates. Mutations in the NPP1 gene have been associated with idiopathic infantile arterial calcification (IIAC), insulin resistance, hypophosphatemic rickets, and ossification of the posterior longitudinal ligament of the spine.

IIAC, a rare autosomal recessive and nearly always fatal disorder, is characterized by calcification of the internal elastic lamina of muscular arteries and stenosis due to myointimal proliferation. There are more than 160 cases of IIAC that have been reported world-wide. The symptoms of the disease most often appear by early infancy, and the disease is lethal by 6 months of age, generally because of ischemic cardiomyopathy, and other complications of obstructive arteriopathy including renal artery stenosis. In more than a dozen reported cases of IIAC, periarticular calcifications of large joints also developed in infancy. Rutsch *et al.* (2003) reported that mutations in ENPP1 are associated with IIAC characterized by the spontaneous periarticular and aortic calcifications in early life and systemic lowering of nucleotide pyrophosphatase/phosphodiesterase activity in the affected individuals.

Johnson et al. (Journal of Bone and Mineral Research, 2003, 18(6): 994-1004) report that extracellular PPᵢ deficiency promoted osteopontin (OPN) deficiency and that correction of OPN deficiency prevents hypercalcification. This document contains no teaching to use soluble NPP1 or OPN molecules therapeutically.

Schmidt (Current Opinion in Drug Discovery & Development 2009, 12(2): 1-12) and Huang (Current Opinion in Biotechnology 2009, 20:692-699) both discuss fusion proteins, *e.g.* Fc fusion proteins, that have been approved for use in the clinic and those that are currently in clinical trials.

Although defects in the NPP1 protein have been implicated in such serious disease as IIAC, there is no treatment available in the art for those who are affected by the disease. Therefore, a dire need exists for an effective and safe composition, formulation and medicament for the treatment of IIAC, insulin resistance, hypophosphatemic rickets, and ossification of the posterior longitudinal ligament of the spine.

### SUMMARY

The present invention and preferred embodiments thereof are set out in the appended claims. More generally, the present disclosure is directed to fusion proteins of truncated domains of NPP1 (*i*.*e*., an NPP1 component) fused to a targeting moiety. The targeting moiety functions to enhance the efficiency in targeting the NPP1 fusion protein to a site clinical or biological importance (*e.g*., site of calcification in a subject that needs lowering of calcification). Without wishing to limit the disclosure to any particular theory or mechanism of operation it is believed that the NPP1 component function to inhibit calcification by enhancing the formation of pyrophosphate (PPi) and/or by cleaving pyrophosphate to produce soluble phosphate (Pi) and/or by increasing the availability of adenosine monophosphate (AMP) and/or adenosine. It is contemplated that the targeting moiety can be attached to the N-terminus and/or the C-terminus of the NPP1 component at any useful position. Additionally, the NPP1 fusion protein disclosed herein can also include one or more of Fc fragment, PEG, polypeptide linker or other additional polypeptides to enhance the enzymatic activity, stability or targeting.

The fusion proteins of the disclosure can be used to treat a wide variety of conditions in a subject. Any condition that can be beneficially treated by the administering of a fusion protein of the invention is included within the scope of the invention. For example, treatment of conditions that can be improved by reducing and/or eliminating one or more calcification structures and/or preventing calcification structures from forming in a subject such as a mammal, for example, a human patient is within the scope of the disclosure. Conditions such as arterial blockage are contemplated for treatment by employing fusion proteins of the invention. In one particularly useful embodiment, the condition to be treated is generalized arterial calcification of infancy also referred to as idiopathic arterial calcification of infancy and arterial media calcification of infancy. Conditions such as insulin resistance, hypophosphatemic rickets, and ossification of the posterior longitudinal ligament of the spine are also contemplated for treatment by employing fusion proteins of the invention.

Fusion proteins of the disclosure can be produced in any useful protein expression system including, without limitation, cell culture (*e.g*., CHO cells, COS cells, HEK203), bacteria such as *Escherichia coli* (*E. coli*) and transgenic animals, including, but no limited to, mammals and avians (*e.g*., chickens, quail, duck and turkey).

The manufacture of pharmaceutical compositions (or pharmaceutical formulations) is well known in the art and such pharmaceutical compositions are contemplated for use in accordance with fusion proteins of the disclosure.

Generally, the dosage of fusion protein administered to a subject will vary depending upon known factors such as age, health and weight of the recipient, type of concurrent treatment, frequency of treatment, and the like. Usually, a dosage of active ingredient (*i.e*., fusion protein) can be between about 0.0001 and about 50 milligrams per kilogram of body weight. Precise dosage, frequency of administration and time span of treatment can be determined by a physician skilled in the art of administration of therapeutic proteins.

### BRIEF DESCRIPTION OF THE DRAWINGS

Fig. 1 illustrates the amino acid sequence of wild-type NPP1 protein. The cytosolic and transmembrane regions are underlined. The potential N-glycosylation sites are in bold. PSCAKE in italics is the start of soluble NPP1 which includes the cysteine rich region.
Fig. 2 illustrates the amino acid sequence of the catalytic domain(s) of NPP1 protein having no target moiety attached ("sssNPP1").

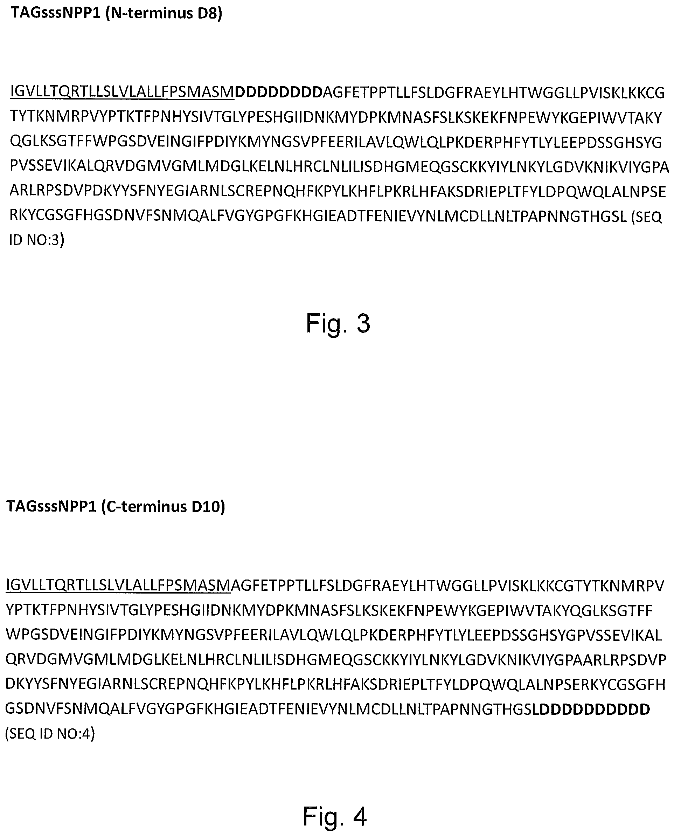

Fig. 3 illustrates the amino acid sequence of TAGsssNPP1. The targeting moiety of eight consecutive aspartic acid residues is fused to the N-terminus of the sssNPP1. The signal peptide is underlined, and the targeting moiety is indicated in bold.
Fig. 4 illustrates the amino acid sequence of TAGsssNPP1 that contains the targeting moiety of ten consecutive aspartic acid residues fused to the C-terminus of the sssNPP1. The signal peptide is underlined, and the targeting moiety is indicated in bold.

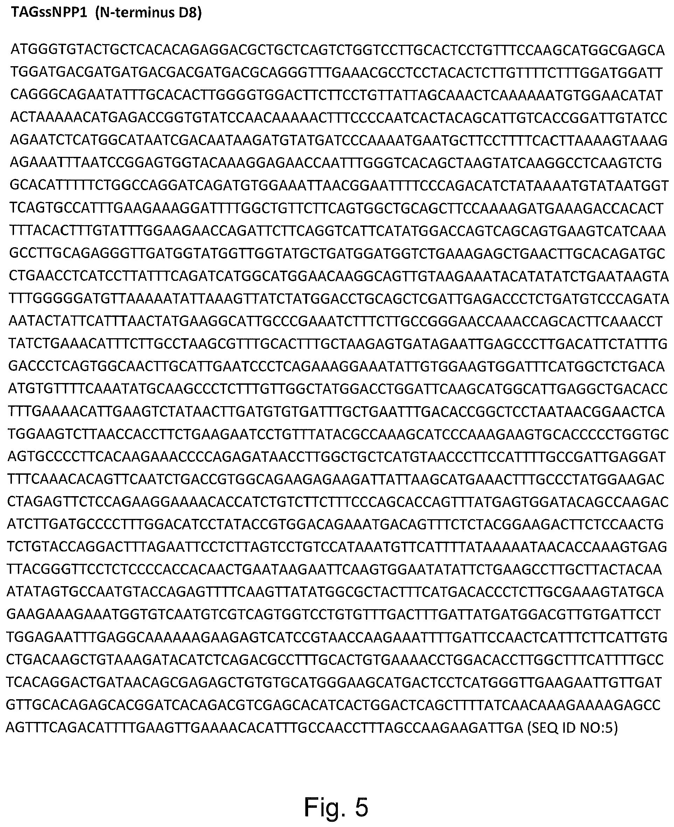

Fig. 5 illustrates the nucleic acid sequence of TAGssNPP1 fusion protein. The targeting moiety of eight consecutive aspartic acid residues is fused to the N-terminus of the ssNPP1.

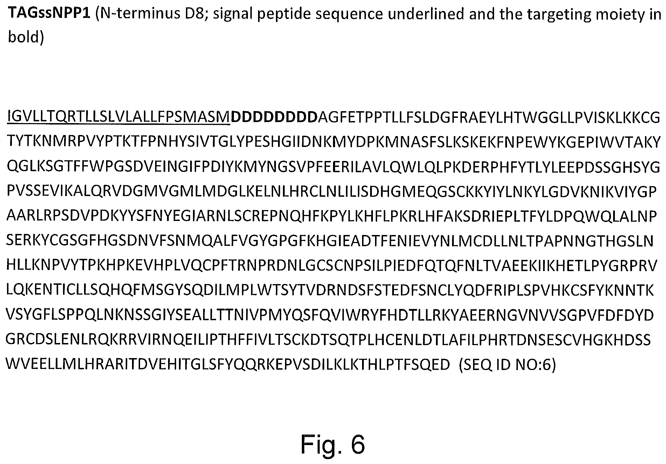

Fig. 6 illustrates the amino acid sequence of TAGssNPP1 fusion protein. The targeting moiety of eight consecutive aspartic acid residues is fused to the N-terminus of the ssNPP1. The signal peptide is underlined, and the targeting moiety is indicated in bold.

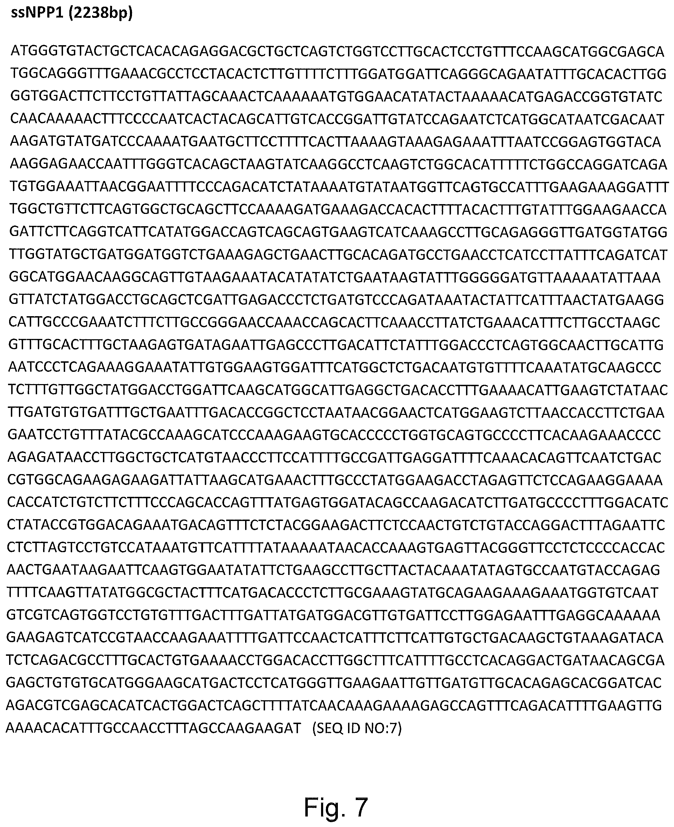

Fig. 7 illustrates the nucleic acid sequence of ssNPP1.

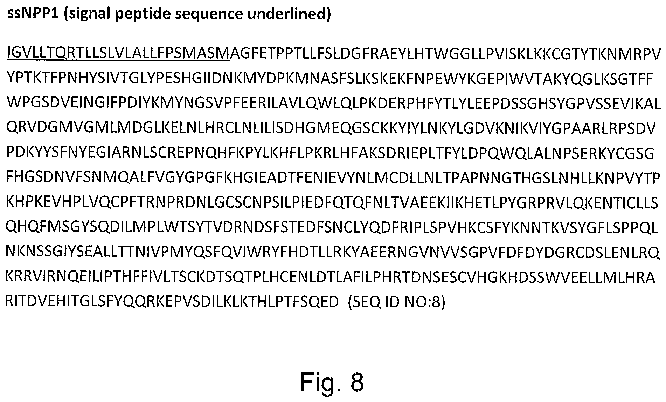

Fig. 8 illustrates the amino acid sequence of ssNPP1. The signal peptide is underlined.
Fig. 9 illustrates the nucleic acid sequence of sNPP1.

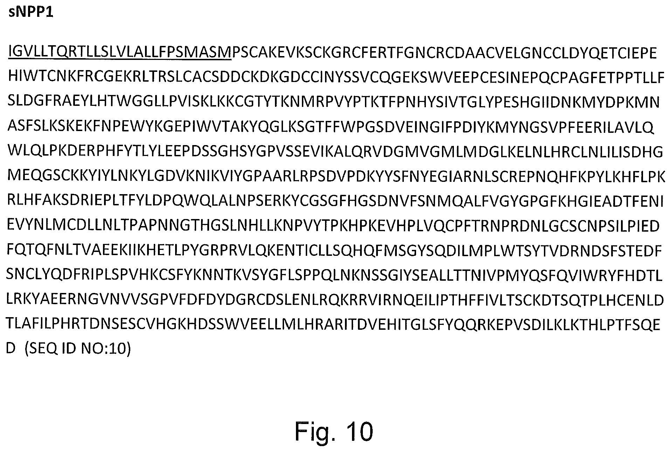

Fig. 10 illustrates the amino acid sequence of sNPP1. The signal peptide is underlined.

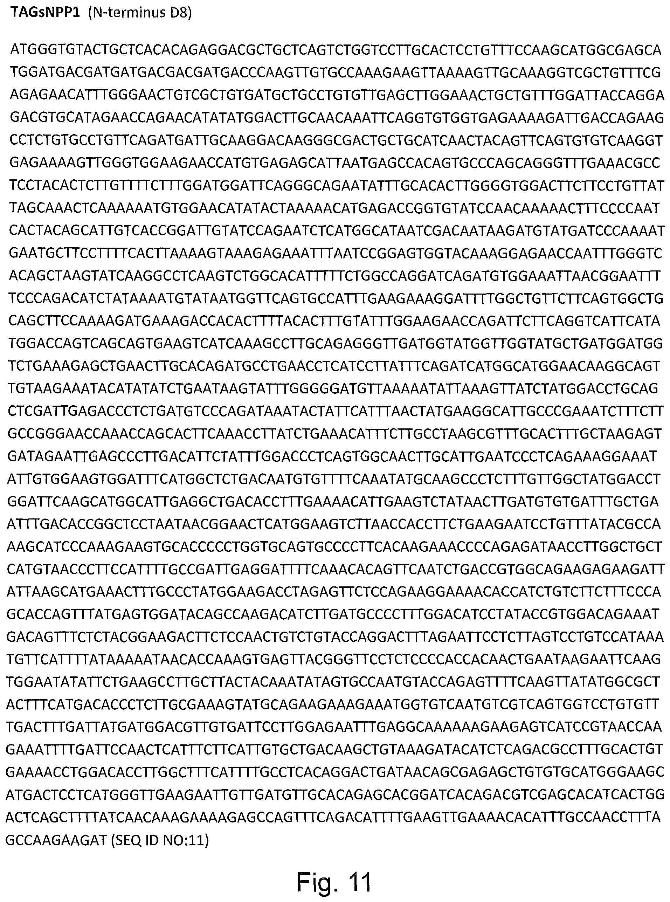

Fig. 11 illustrates the nucleic acid sequence of TAGsNPP1. The targeting moiety of eight consecutive aspartic acid residues is fused to the N-terminus of the sNPP1.

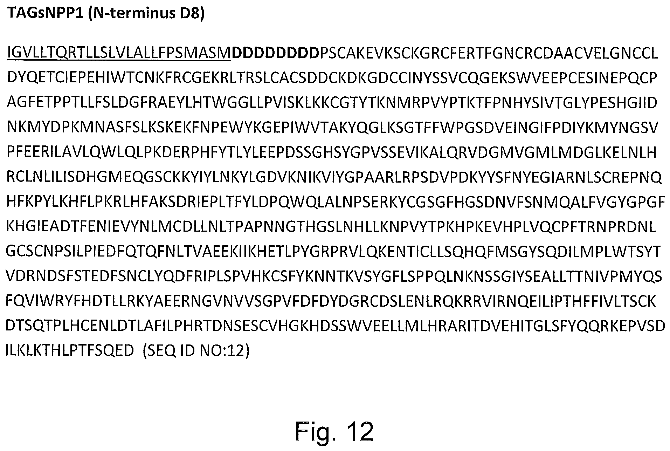

Fig. 12 illustrates the amino acid sequence of TAGsNPP1. The targeting moiety of eight consecutive aspartic acid residues is fused to the N-terminus of the ssNPP1. The signal peptide is underlined, and the targeting moiety is indicated in bold.

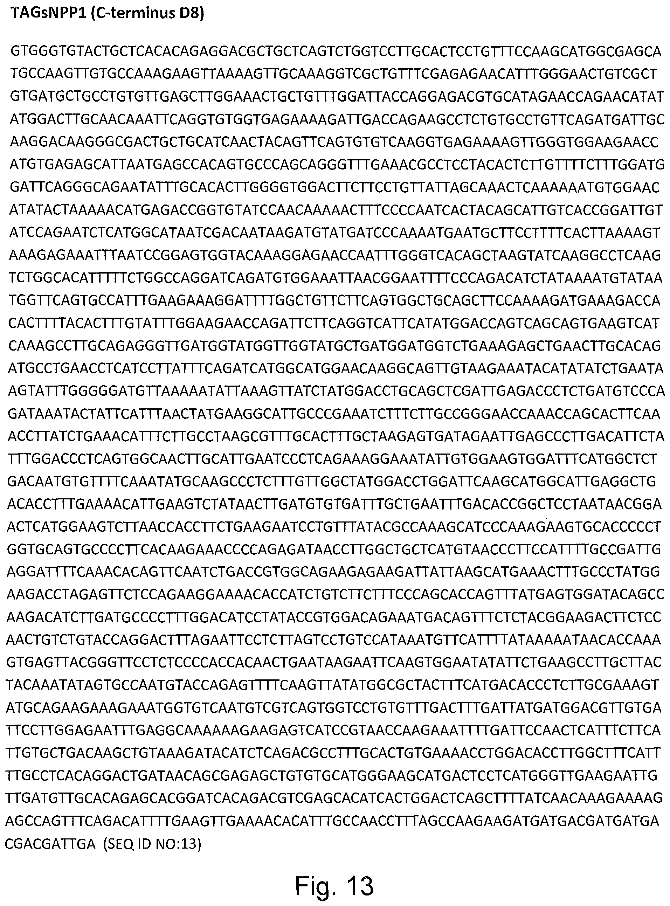

Fig. 13 illustrates the nucleic acid sequence of TAGsNPP1. The targeting moiety of eight consecutive aspartic acid residues is fused to the C-terminus of the sNPP1.

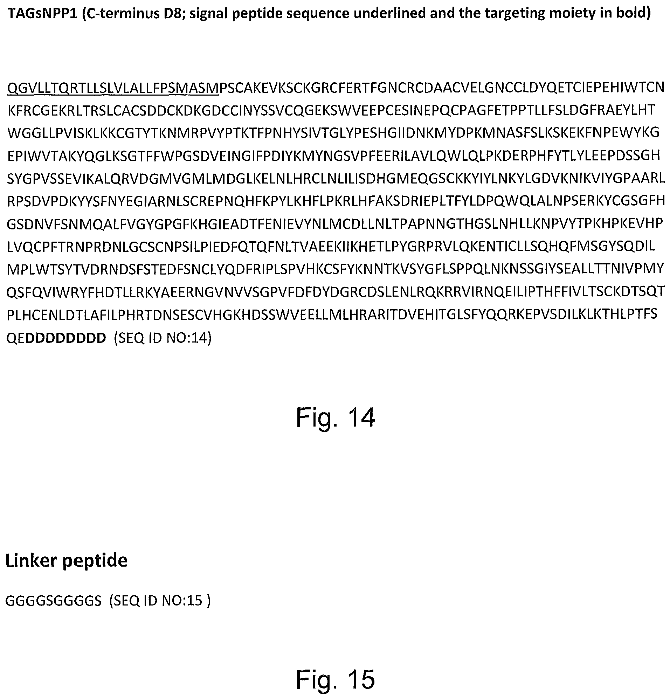

Fig. 14 illustrates the amino acid sequence of TAGsNPP1. The targeting moiety of eight consecutive aspartic acid residues is fused to the N-terminus of the sNPP1. The signal peptide is underlined, and the targeting moiety is indicated in bold.
Fig. 15 illustrates the amino acid sequence of a linker peptide.

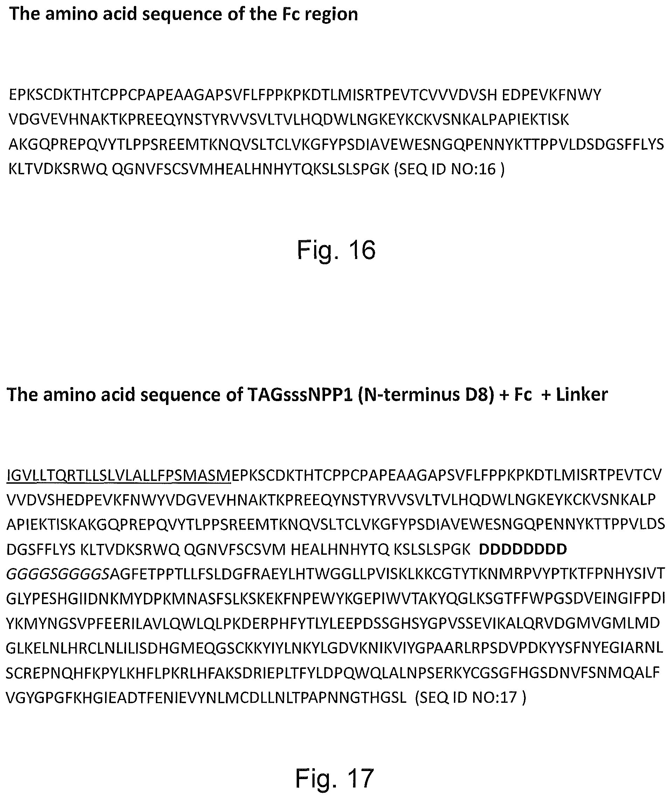

Fig. 16 illustrates the amino acid sequence of an immunoglobulin Fc segment.
Fig. 17 illustrates the amino acid sequence of TAGsssNPP1 which contains the targeting moiety of eight consecutive aspartic acid residues fused to the N-terminus of the sssNPP1 via a peptide linker. The Fc segment is fused to N-terminus of the target moiety. The signal peptide is underlined and the targeting moiety is indicated in bold. The peptide linker is in italics.

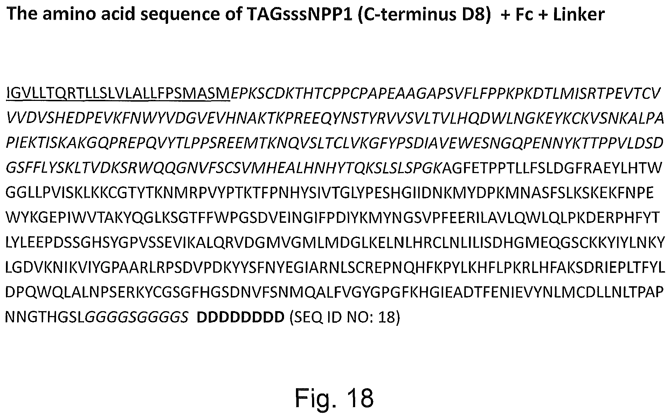

Fig. 18 illustrates the amino acid sequence of TAGsssNPP1 which contains the targeting moiety of eight consecutive aspartic acid residues fused to the C-terminus of the sssNPP1 via a peptide linker. The Fc segment is fused to N-terminus of the sssNPP1. The signal peptide is underlined and the targeting moiety is indicated in bold. The peptide linker is in italics.
Fig. 19 is a schematic representation of an expression vector (*i.e*., pTT22) containing a TAGsNPP1 construct. The targeting moiety of eight consecutive aspartic acid residues fused to the C-terminus of the sNPP1.

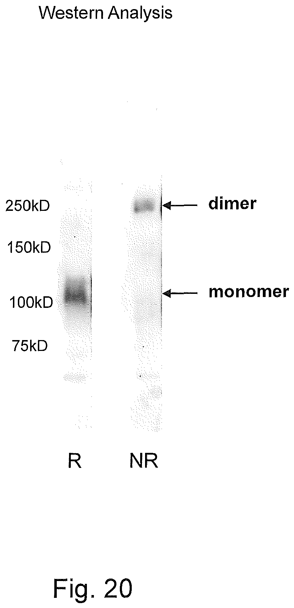

Fig. 20 illustrates Western blot analysis of TAGsNPP1. Reducing condition; NR, non-reducing condition.
Fig. 21 demonstrates the enzymatic activity of TAGsNPP1 produced and isolated from HEK293 cells.

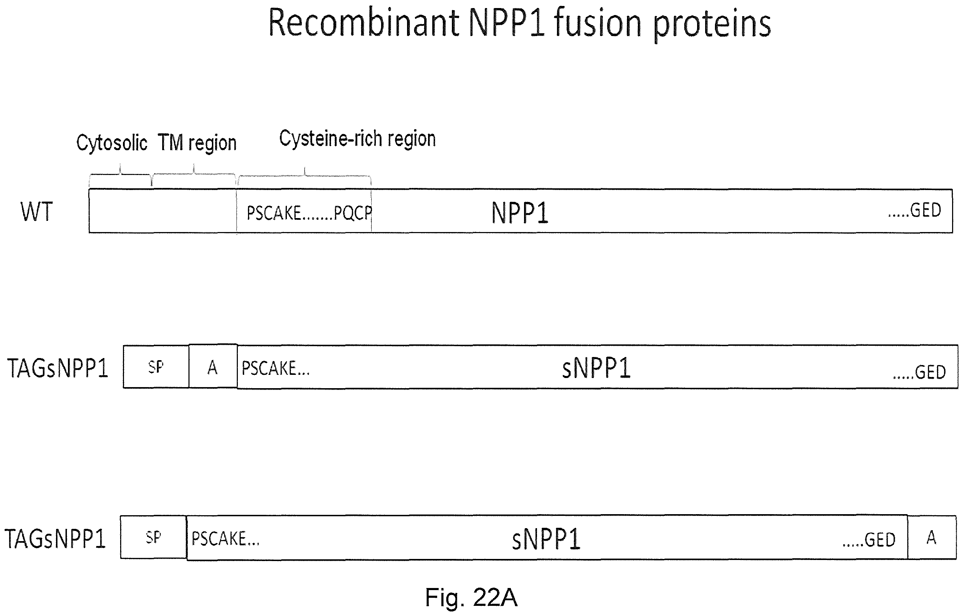

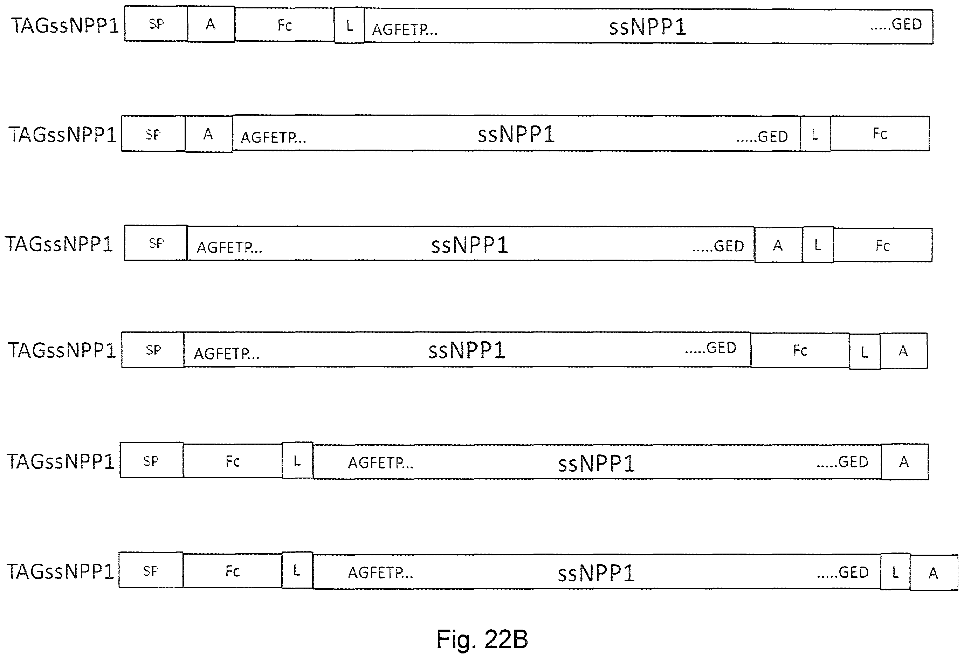

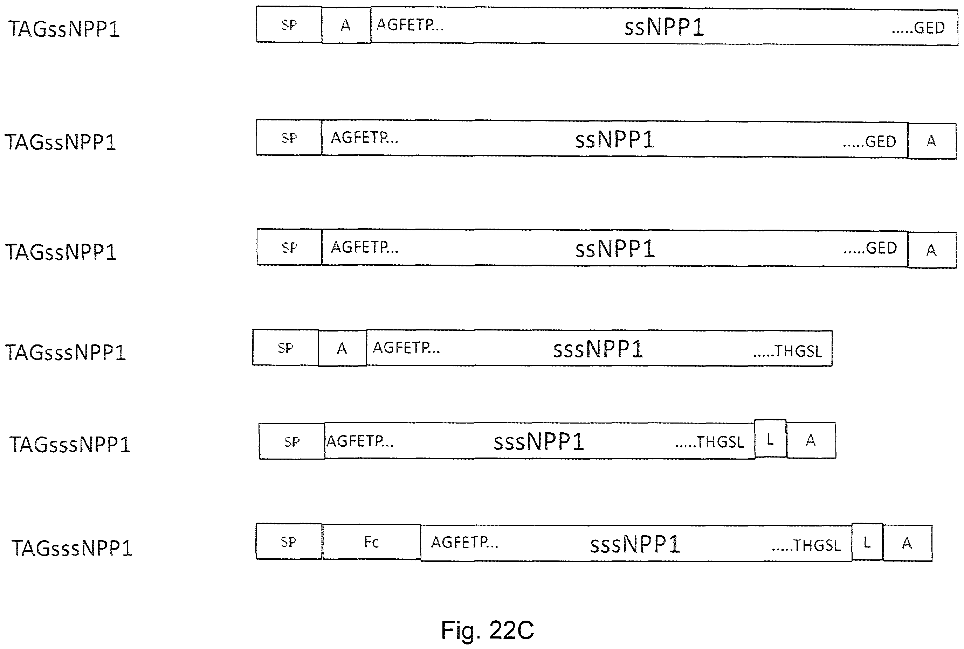

Figs. 22A-22C illustrate schematics of TAGNPP1 fusion protein constructs described herein.
Fig. 23 demonstrates calicification levels of human aortic smooth muscle cells treated with soluble sNPP1 (WT), TAGsNPP1 (D8 fused to sNPP1 C-terminus), and sNPP1-Fc.

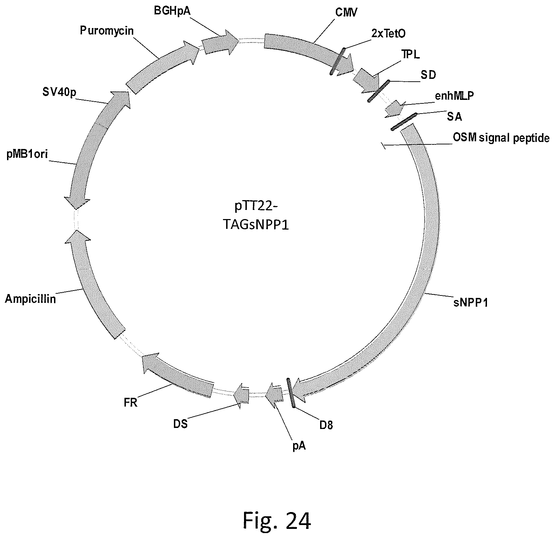

Fig. 24 depicts a schematic representation of an expression vector (*i*.*e*., pTT22) containing a TAGsNPP1 construct. The targeting moiety of eight consecutive aspartic acid residues (D8) fused to the C-terminus of the sNPP1.

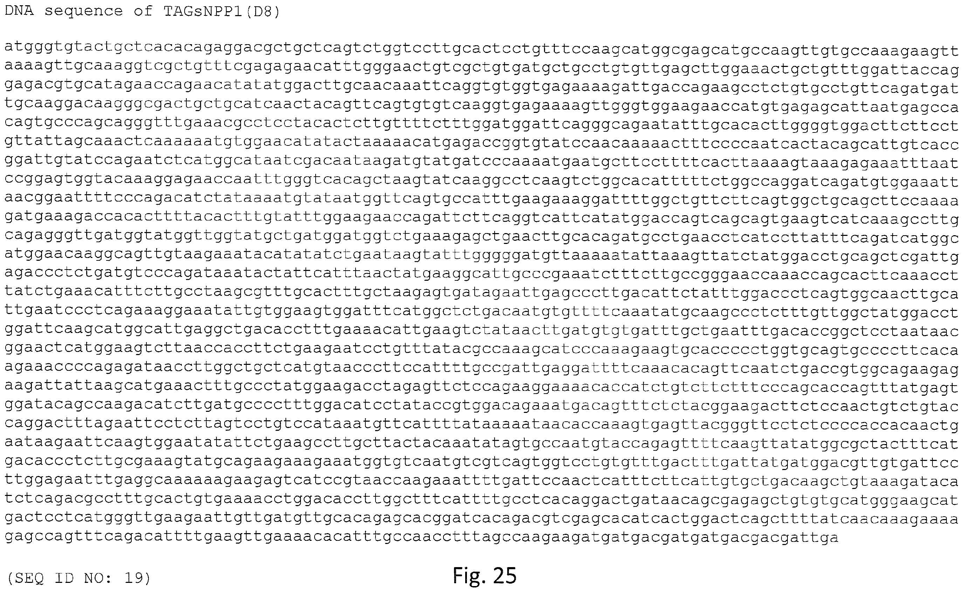

Fig. 25 depicts the nucleic acid sequence of a TAGsNPP1 fusion protein.
Fig. 26 depicts the amino acid sequence of a TAGsNPP1 fusion protein.

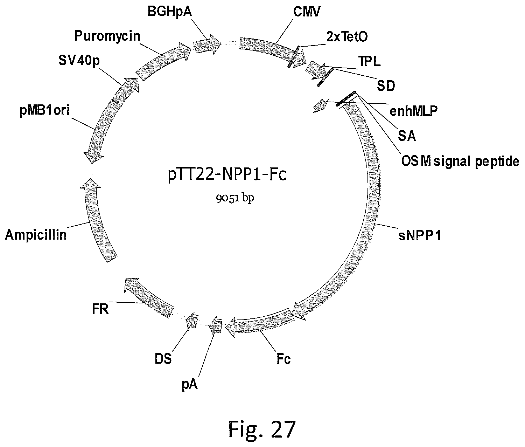

Fig. 27 depicts a schematic representation of an expression vector (*i.e*., pTT22) containing a sNPP1-Fc fusion construct.

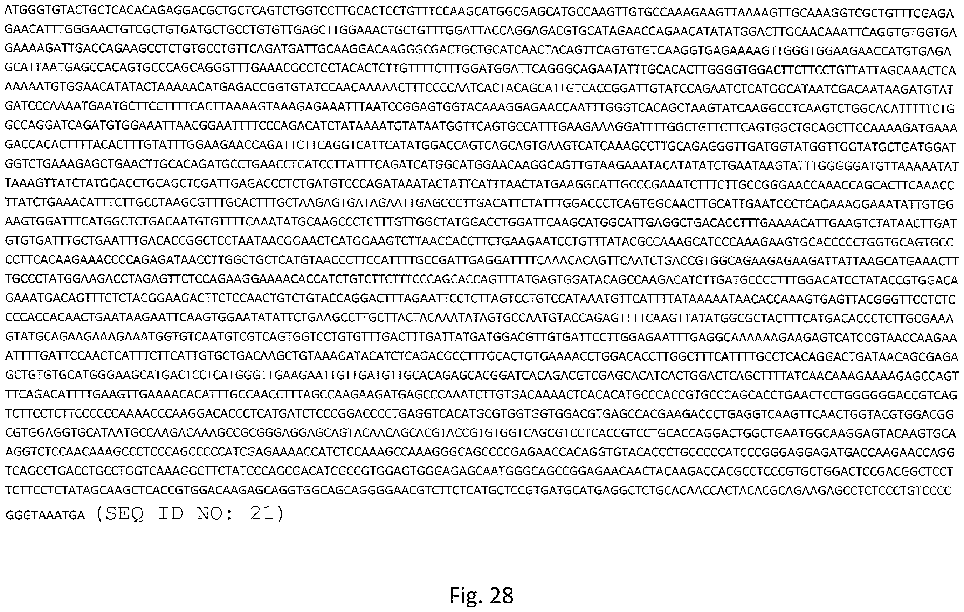

Fig. 28 depicts the nucleic acid sequence of a sNPP1-Fc fusion protein.
Fig. 29 depicts the amino acid sequence of a sNPP1-Fc fusion protein.

### DETAILED DESCRIPTION

The present disclosure provides novel human NPP1 fusion proteins that are soluble and contain truncated and biologically active domain(s) of NPP1 (*i.e*., NPP1 components that contain at least one extracellular catalytic domain of naturally occurring NPP1 for the pyrophosphatase and/or phosphodiesterase activity) and one or more targeting moieties (*i.e*., "TAG"). The NPP1 fusion proteins of the presentdisclosure comprise at least the NPP1 domain essential to carry out the pyrophosphatase and/or phosphodiesterase activity. Accordingly, the disclosure features isolated fusion proteins comprising the amino acid residues A205 through L591 of SEQ ID NO:1 fused to one or more targeting moieties. The targeting moiety can be recombinantly fused or chemically bonded (*e.g*., covalent bond, ionic bond, hydrophobic bond and Van der Waals force) to the NPP1 component by methods well known in the art and direct the NPP1 component to a certain target site where the attached NPP1 component will have a desirable effect (*e.g*., catalysis of a reaction such as solubilizing a substrate such as PPi or preventing the formation of a substrate such as PPi) in a subject to which the fusion protein of the present disclosure is administered.

### TAGNPP1s

All NPP1 fusion proteins ("TAGNPP1s") of the present disclosure have the N-terminal cytosolic and the transmembrane domains of the naturally occurring human NPP1 removed. Optionally, TAGNPP1s fusion proteins of the present disclosure can also contain C-terminal truncation of wild-type NPP1 in various lengths. The amino acid sequence of full-length wild-type NPP1 is set forth in SEQ ID NO: 1.

In one instance, the fusion protein contains a polypeptide comprising the amino acid residues A205 through L591 of SEQ ID NO:1 ("sssNPP1") fused a TAG on either the N- or C-terminus of the polypeptide ("TAGsssNPP1"). In one instance, the fusion protein comprises a polypeptide comprising the amino acid residues A205 through D925 of SEQ ID NO:1 ("ssNPP1") fused to a TAG on either the N- or C-terminus ("TAGssNPP1"). In one instance, the fusion protein comprises a polypeptide comprising the amino acid residues P99 through D925 of SEQ ID NO:1 ("sNPP1") fused to a TAG on either the N- or C- terminus of the polypeptide ("TAGsNPP1"). Also disclosed herein is any consecutive fragment of sNPP1 that comprises at least the amino acid resides A205 through L591 of SEQ ID NO:1 and the polypeptide fragment is fused to a TAG on either the N- or C-terminus.

When expressed in a cell culture or transgenic animal, the TAGNPP1 fusion proteins can further comprise a signal peptide (or leader sequence) at its N-terminus. The signal peptide co-translationally or post-translationally directs transport of the TAGNPP1 fusion proteins through the subcellular organelles of the cell expressing the TAGNPP1 fusion proteins and, thereby determining the post-translational modification of the TAGNPP1 fusion proteins. It is to be understood that because the signal peptide is cleaved at the co-translational or post-translational stage of the fusion protein, the TAGNNP1 fusion proteins are generally devoid of the signal peptide when once secreted and isolated. Accordingly, in the instances that are directed to the nucleic acid sequences encoding the TAGNPP1 fusion proteins are described, the leader sequences are also contemplated as used in the present disclosure. For example, the nucleotide sequence set forth in SEQ ID NO:2 contains an example of the leader sequence for TAGNNP1 at its 5' end.

Each of the fusion proteins disclosed herein is contemplated with one or more targeting moieties ("TAG"). The TAG component according to the present disclosure comprises four or more negatively charged amino acids such as aspartic acid and glutamic acid. The TAG component can be a stretch of negatively charged amino acid residues, for example, aspartic acids and/or glutamic acids that are between about 4 and about 20 amino acid residues in length. The TAG can be fused to either the N- or C-terminus of the NPP1 component. The TAG can be also fused to both N- and C-termini of the NPP1 component. Accordingly, the amino acid sequence of the TAGsNPP1 fusion protein, for example, includes PSCAKE through the C-terminus end of the NPP1 component and one or more targeting moieties (*e.g*., polyglutamic acid tag or polyaspartic acid tag) at the N- and/or C-terminus end of the NPP1 component. The fusion protein comprising the NPP1 component having the TAG fused to the C-terminus is a particularly useful instance. In one very specific instance, TAG having a stretch of eight aspartic acids is employed as can be seen in the exemplary sequences for TAGsNPP1 and TAGssNPP1 in Figures, though any useful number of negatively charged amino acid residues (*e.g*., 4, 5, 6, 7, 9, 10, 11, 12, 13, 14, 15, 16, 17, or 18) can be used in accordance with the disclosure. The TAG component is indicated as "A" in Figures.

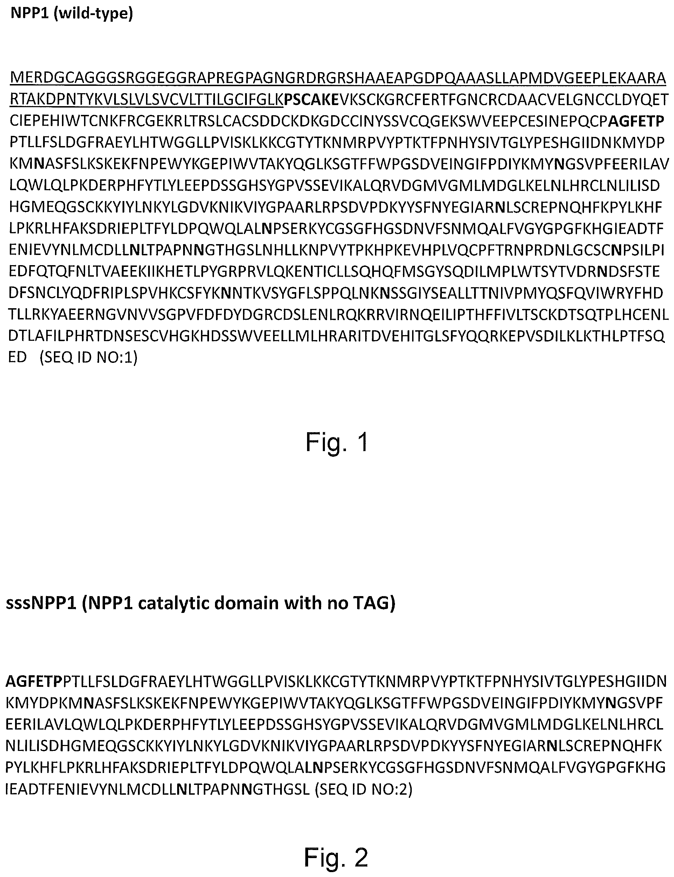

The disclosure also encompasses polynucleotides which encode various TAGNPP1 fusion proteins described herein. Accordingly, any nucleic acid sequence which encodes the amino acid sequence of any TAGNPP1 fusion protein can be used to generate recombinant molecules which express the corresponding TAGNPP1 fusion protein. In a particular instance, the disclosure encompasses the polynucleotide comprising the nucleic acid sequence of SEQ ID NO:2 as shown in FIG. 2.

Within certain specific instances, the fusion protein comprises multiple polypeptides as described herein, or that comprises at least one polypeptide as described herein and an unrelated sequence. Certain preferred polypeptide can, for example, assist in dimerization and stability or minimize aggregation of the fusion proteins. For example, the additional polypeptide can be the Fc region of the immunoglobulin G1 to increase stability in serum. The use of the Fc segment is well known in the art and described in U.S. Pat. No. 7,902,151; and U.S. Pat. No. 7,858,297. The cysteine rich region of wild-type NPP1 (*i.e*., PSCAKE through NEPQCP; the amino acid sequence from P99 to P204 of SEQ ID NO:1) can be employed to facilitates dimerization of the TAGNPP1 fusion proteins.

In another instance, the polyethylene glycol (PEG) can be conjugated to the TAGNPP1 fusion proteins. Other polypeptides may be selected so as to minimize aggregation and immunogenicity, to increase the solubility of the protein, or to enable the protein to be targeted to desired sites of clinical or biological importance.

TAGNPP1 can be also fused or conjugated to an appropriate polypeptide linker or other sequence for ease of identification, synthesis, or purification of the fusion protein, or to better preserve the native structure of the NPP1 component which can enhance the activity and targeting of the TAGNPP1. Specifically, a peptide linker sequence can be employed to separate the first and the second polypeptide components by a distance sufficient to ensure that each polypeptide folds into its secondary and tertiary structures. Such a peptide linker sequence is incorporated into the fusion protein between the NPP1 component and the TAG component using standard techniques well known in the art. Suitable peptide linker sequences may be chosen based on their ability to adopt a flexible extended conformation and their inability to adopt a secondary structure that could interact with functional portion on the NPP1, TAG or other secondary polypeptides described herein (*e.g*., Fc). Preferred peptide linker sequences contain Gly, His, Asn and Ser residues. The useful peptide linkers include, without limitation, poly-Gly, poly-His, poly-Asn, or poly-Ser. Other near neutral amino acids, such as Thr and Ala can be also used in the linker sequence. Amino acid sequences which can be usefully employed as linkers include those disclosed in Maratea et al., Gene 40:39-46, 1985; Murphy et al., Proc. Natl. Acad Sci. USA 83:8258-8262, 1986; U.S. Pat. No. 4,935,233 and U.S. Pat. No. 4,751,180. The linker sequence may be from 1 to about 20 amino acid residues in length. Preferably, the polypeptide linker is between about 8 and about 12 amino acids in length. In a preferred instance, the peptide linker used in the disclosure is GGGGSGGGGS (SEQ ID NO:15), although any functional combination of Gly, Ser, His, or Asn can be employed.

Fusion proteins can also comprise a TAGNPP1 of the present disclosure together with an unrelated polypeptide. Preferably the unrelated polypeptide is capable of enhancing the targeting of the fusion protein to the site of clinical or biological importance (*e.g*., site of calcification). For example, peptides that have high affinity to the bone are described in U.S. Pat. No. 7,323,542.

TAGNPP1 can be prepared using standard methods, including recombinant techniques or chemical conjugation well known in the art. Techniques useful for isolating and characterizing the nucleic acids and proteins of the present disclosure are well known to those of skill in the art and standard molecular biology and biochemical manuals can be consulted to select suitable protocols for use without undue experimentation. See, for example, Sambrook et al, 1989, "Molecular Cloning: A Laboratory Manual," 2nd ed., Cold Spring Harbor. Briefly, DNA sequences encoding the polypeptide components can be assembled separately, and ligated into an appropriate expression vector. For example, the 3' end of the DNA sequence encoding the NPP1 component is ligated, with or without a peptide linker, to the 5' end of a DNA sequence encoding the second polypeptide component such as TAG PEG, or Fc so that the reading frames of the sequences are in phase. This permits translation into a single fusion protein that retains the biological activity of both component polypeptides. The ligated DNA sequences are operably linked to suitable transcriptional or translational regulatory elements including a promoter. The regulatory elements responsible for expression of DNA are located only 5' to the DNA sequence encoding the first polypeptide such as the leader sequence encoding a signal peptide. Similarly, stop codons required to end translation and transcription termination signals are only present 3' to the DNA sequence encoding the second polypeptide.

The disclosure also encompasses TAGNPP1 variants. A preferred TAGNPP1 variant is one having 80%, 85%, 90%, 95% and more preferably 96% amino acid sequence identity to the amino acid sequence A205 through L591 of SEQ ID NO:1. A most preferred TAGNPP1 variant is one having at least 97% amino acid sequence identity to amino acid sequence A205 through L591 of SEQ ID NO:1.

The disclosure also relates to a polynucleotide sequence comprising the complement of SEQ ID NO:2 or variants thereof. In addition, the present disclosure also features polynucleotide sequences which hybridize under stringent conditions to SEQ ID NO:2 and whose the antisense sequence is 85%, 90%, 95%, 97%, 98%, or 99% identical to SEQ ID NO:2. Hybridization conditions are based on the melting temperature (Tm) of the nucleic acid binding complex or probe, as taught in Wahl, G. M. and S. L. Berger (1987; Methods Enzymol. 152:399-407) and Kimmel, A. R. (1987; Methods Enzymol. 152:507-511), and can be used at a defined stringency.

The disclosure additionally contemplates nucleic acid sequences encoding polypeptides, oligonucleotides, peptide nucleic acids (PNA), fragments, portions or antisense molecules thereof.

Although nucleotide sequences which encode TAGNPP1 and its variants are preferably capable of hybridizing to the nucleotide sequence of the TAGNPP1 under appropriately selected conditions of stringency, it can be advantageous to produce nucleotide sequences encoding TAGNPP1 or its derivatives possessing a substantially different codon usage. Codons can be selected to increase the rate at which expression of the peptide occurs in a particular prokaryotic or eukaryotic host in accordance with the frequency with which particular codons are utilized by the host. Other reasons for substantially altering the nucleotide sequence encoding TAGNPP1 and its derivatives without altering the encoded amino acid sequences include the production of RNA transcripts having more desirable properties, such as a greater half-life.

Altered nucleic acid sequences encoding TAGNPP1 which are encompassed by the disclosure include deletions, insertions, or substitutions of different nucleotides resulting in a polynucleotide that encodes the same or a functionally equivalent of TAGNPP1. The encoded protein can also contain deletions, insertions, or substitutions of amino acid residues which produce a silent change and result in a functionally equivalent TAGNPP1. Deliberate amino acid substitutions can be made on the basis of similarity in polarity, charge, solubility, hydrophobicity, hydrophilicity, and/or the amphipathic nature of the residues as long as the biological activity of TAGNPP1 is retained. For example, positively charged amino acid residues include Lys and Arg; negatively charged amino acid residues include Asp and Glu; and amino acids with uncharged polar head groups having similar hydrophilicity can include Leu, Ile, and Val; Gly and Ala; Asp and Gln; Ser and Thr; Phe and Tyr.

### Expression vector

Methods which are well known to those skilled in the art can be used to construct expression vectors containing sequences encoding TAGNPP1 and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York, N.Y..

A variety of expression vector/host systems can be utilized to contain and express sequences encoding TAGNPP1. These include, but are not limited to, microorganisms such as bacteria transformed with recombinant bacteriophage, plasmid, or cosmid DNA expression vectors; yeast transformed with yeast expression vectors; insect cell systems infected with virus expression vectors (*e.g*., baculovirus) or with bacterial expression vectors (*e.g*., Ti or pBR322 plasmids); or animal cell systems (*e.g*., pTT22 vector).

The control elements or regulatory sequences can includes those non-translated regions of the vector-enhancers, promoters, 5' and 3' untranslated regions-which interact with host cellular proteins to carry out transcription and translation. Such elements can vary in their strength and specificity. Depending on the vector system and host utilized, any number of suitable transcription and translation elements, including, tissue-specific, constitutive and inducible promoters, can be used. For example, when cloning in bacterial systems, inducible promoters such as the hybrid lacZ promoter of the Bluescript™ phagemid (Stratagene, LaJolla, California) or pSport1™ plasmid (Gibco BRL) and the like can be used. In mammalian cell systems, promoters from mammalian genes or from mammalian viruses are preferred. If it is necessary to generate a cell line that contains multiple copies of the sequence encoding TAGNPP1, vectors based on SV40 or EBV can be used with an appropriate selectable marker. When an avian expression system is used, suitable vectors for expression various TAGNPP1 constructs are described in U.S. Pat. No. 6,730,822; U.S. Pat. No. 6,825,396; U.S. Pat. No. 6,875,588; U.S. Pat. No. 7,294,507; U.S. Pat. No. 7,521,591; U.S. Pat. No. 7,534,929; and U.S. patent application Ser. No. 11/376,023. Briefly, when an avian expression system is employed to express TAGNPP1, suitable oviduct-specific promoters, for example, and without limitation, ovomucoid promoters, ovalbumin promoters, lysozyme promoters, conalbumin promoters, ovomucin promoters, ovotransferrin promoters and functional portions of each of these promoters are contemplated. Suitable nonspecific promoters can include, for example and without limitation, cytomegalovirus (CMV) promoters, MDOT promoters and rous-sarcoma virus (RSV) promoters, murine leukemia virus (MLV) promoters, mouse mammary tumor virus (MMTV) promoters and SV40 promoters and functional portions of each of these promoters. Non-limiting examples of other promoters which can be useful include, without limitation, Pol III promoters (for example, type 1, type 2 and type 3 Pol III promoters) such as HI promoters, U6 promoters, tRNA promoters, RNase MPR promoters and functional portions of each of these promoters. Typically, functional terminator sequences are selected for use in accordance with the promoter that is employed.

### Host Cells

The present disclosure includes the production of soluble TAGNPP1 in a transgenic avian (e.g., transgenic chicken) system as is well known in the art, for example, in U.S. Patent No. 7,534,929. Production in the avian system (*e.g*., in the avian oviduct) of an NPP1 component with or without to a targeting moiety (*e.g*., ssNPP1, sNPP1, TAGsNPP1 and TAGssNPP1) is within the scope of the disclosure. Furthermore, TAGNPP1 produced in any useful protein expression system including, without limitation, transgenic avians, transgenic mammal, cell culture (*e.g*., CHO cells, HEK293 cells, and COS cells), bacteria such as *E. coli,* transgenic animals such as mammals and avians (*e.g*., chickens, quail, duck and turkey) and in plant systems including duck weed, is contemplated herein.

A host cell strain can be chosen for its ability to modulate the expression of the inserted sequences or to process the expressed TAGNPP1s in the desired fashion. Such modifications of the polypeptide of TAGNNP1 include, without limitation, acetylation, carboxylation, sialylation, glycosylation, phosphorylation, lipidation, and acylation. Different host cells such as CHO, COS, HeLa, MDCK, HEK293, and W138, which have specific cellular machinery and characteristic mechanisms for such post-translational activities, can be chosen to ensure the correct modification and processing of the fusion protein of the present disclosure. Avian tumor cell line is also contemplated as a host cell for expressing the polypeptide of the present disclosure. Examples of useful avian cell lines (*e.g*., an avian oviduct tumor cell line) which can be employed are described in U.S. Pat. Publication No. 2009/0253176.

### Production of TAGNPP1

TAGNPP1 can be produced using any of a variety of well-known techniques. TAGNPP1 encoded by DNA sequences as described above can be readily prepared from the DNA sequences using any of a variety of expression vectors described herein or well known to those of ordinary skill in the art. Expression can be achieved in any appropriate host cell that has been transformed or transfected with an expression vector containing a DNA molecule that encodes a recombinant polypeptide of the present disclosure. Supernatants from suitable host/vector systems which secrete recombinant fusion protein or polypeptide into culture media can be first concentrated using a commercially available filter. Following concentration, the concentrate can be applied to a suitable purification matrix such as an affinity matrix or an ion exchange resin. One or more reverse phase HPLC steps can be employed to further purify a recombinant polypeptide.

For high-yield production of recombinant proteins, stable expression is preferred. Cell lines stably expressing TAGNPP1 can be transformed using expression vectors which contain viral origins of replication and/or endogenous expression elements and/or a selectable marker gene on the same or on a separate vector. Following the introduction of the vector, cells can be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells can be proliferated using tissue culture techniques appropriate to the cell type. Methods of producing exogenous protein in mammalian cell lines are well known in the art. Illustrative examples of this and other aspects and instances of the present disclosure for the production of heterologous polypeptides such as TAGNPP1 fusion proteins in avian cells are fully disclosed in U.S. patent application serial No. 09/877,374, filed Jun. 8, 2001, published as U.S. 2002/0108132-A1 on Aug. 8, 2002, and U.S. patent application serial No. 10/251,364, filed Sep. 18, 2002. Examples of producing exogenous proteins in avian tumor cell lines are also described in U.S. Pat. Publication No. 2009/0253176.

Specifically contemplated is the production of the TAGNPP1 proteins disclosed herein in a transgenic avian system. In one particularly useful instance, the disclosure is drawn to the production of TAGNPP1 which can be produced in the oviduct of a transgenic avian, such as a chicken. Examples of producing exogenous proteins in transgenic avian expression system are also described in U.S. Pat. No. 6,730,822. Briefly, a suitable avian vector described above that contains a nucleic acid sequence encoding a TAGNPP1 fusion protein, operably linked to a tissue-specific or constitutive promoter that drives expression of the encoding sequence in the chicken oviduct are introduced into chicken stage X embryonic cells. The transformed embryonic cells are incubated under conditions conducive to hatching live chicks. Live chicks are nurtured into a mature chimeric chicken which are mated with a non-transgenic chicken naturally or via artificial insemination. A transgenic chicken is identified by screening progeny for germ line incorporation of the protein encoding sequence. The transgenic progeny can be mated with another transgenic or a non-transgenic chicken to produce eggs containing the TAGNPP1 fusion protein. The TAGNPP1 is then isolated and purified by methods well known in the art. Accordingly, the disclosure provides recombinant TAGNPP1 fusion proteins that have been produced by transgenic avians.

### Pharmaceutical Composition

The present disclosure also features pharmaceutical compositions comprising isolated and substantially purified TAGNPP1 or a pharmaceutically acceptatable salt thereof. Pharmaceutical composition of the present disclosure can also include a pharmaceutically acceptable carrier or expient therefor. Compositions comprising such carriers, including composite molecules, are formulated by well-known conventional methods (*see*, for example, Remington's Pharmaceutical Sciences, 14th Ed., Mack Publishing Co., Easton, Pa.). The carrier may comprise a diluent. In one instance, the pharmaceutical carrier can be a liquid and the fusion protein may be in the form of a solution. The pharmaceutical carrier can be wax, fat, or alcohol. In another instance, the pharmaceutically acceptable carrier may be a solid in the form of a powder, a lyophilized powder, or a tablet. In one instance, the carrier may comprise a liposome or a microcapsule.

The pharmaceutical compositions can be in the form of a sterile lyophilized powder for injection upon reconstitution with a diluent. The diluent can be water for injection, bacteriostatic water for injection, or sterile saline. The lyophilized powder may be produced by freeze drying a solution of the fusion protein to produce the protein in dry form. As is known in the art, the lyophilized protein generally has increased stability and a longer shelf life than a liquid solution of the protein.

### Definitions:

As used herein, the term "acceptable" with respect to a formulation, composition or ingredient, as used herein, means having no persistent detrimental effect on the general health of the subject being treated.

As used herein, the term "administration" or "administering" refers to providing a fusion protein of the disclosure to a subject in need of treatment.

"Alterations," as used herein, comprise any alteration in the sequence of polynucleotides encoding TAGNPP1 including deletions, insertions, and point mutations that may be detected using hybridization assays.

The term "animal" is used herein to include all vertebrate animals, including avians and mammals such as rat, mouse and human. It also includes an individual animal in all stages of development, including embryonic and fetal stages.

Where "amino acid sequence" is recited herein to refer to an amino acid sequence of a fusion protein molecule, "amino acid sequence" and like terms, such as "polypeptide" or "protein" are not meant to limit the amino acid sequence to the complete amino acid sequence associated with the recited protein or polypeptide molecule.

The term "avian" as used herein refers to any species, subspecies or strain of organism of the taxonomic class ava, such as, but not limited to, such organisms as chicken, turkey, duck, goose, quail, pheasants, parrots, finches, hawks, crows and ratites including ostrich, emu and cassowary. The term includes the various known strains of Gallus gallus, or chickens, (for example, White Leghorn, Brown Leghorn, Barred-Rock, Sussex, New Hampshire, Rhode Island, Ausstralorp, Minorca, Amrox, California Gray, Italian Partridge-colored), as well as strains of turkeys, pheasants, quails, duck, ostriches and other poultry commonly bred in commercial quantities.

The phrase "based on" or "derived from" as in a retroviral vector being based on or derived from a particular retrovirus or based on a nucleotide sequence of a particular retrovirus mean that the genome of the retroviral vector contains a substantial portion of the nucleotide sequence of the genome of the particular retrovirus. The substantial portion may be a particular gene or nucleotide sequence such as the nucleotide sequence encoding the gag, pol and/or env proteins or other structural or functional nucleotide sequence of the virus genome such as sequences encoding the LTRs or may be substantially the complete retrovirus genome, for example, most (e.g., more than 60% or more than 70% or more than 80% or more than 90%) or all of the retrovirus genome, as will be apparent from the context in the specification as the knowledge of one skilled in the art. Examples of retroviral vectors that are based on or derived from a retrovirus are the NL retroviral vectors (*e.g*., NLB) which are based on the ALV retrovirus as disclosed in Cosset et al., Journal of Virology (1991) vol 65, p 3388-3394.

The term "biologically active," as used herein, refers to a fusion protein having structural, regulatory, or biochemical functions of pyrophosphatase/phosphodiesterase of a naturally occurring NPP1 protein.

The term "construct," as used herein, refers to a linear or circular nucleotide sequence such as DNA that has been assembled from more than one segments of nucleotide sequence which have been isolated from a natural source or have been chemically synthesized, or combinations thereof.

The term "complementary," as used herein, refers to two nucleic acid molecules that can form specific interactions with one another. In the specific interactions, an adenine base within one strand of a nucleic acid can form two hydrogen bonds with thymine within a second nucleic acid strand when the two nucleic acid strands are in opposing polarities. Also in the specific interactions, a guanine base within one strand of a nucleic acid can form three hydrogen bonds with cytosine within a second nucleic acid strand when the two nucleic acid strands are in opposing polarities. Complementary nucleic acids as referred to herein, may further comprise modified bases wherein a modified adenine may form hydrogen bonds with a thymine or modified thymine, and a modified cytosine may form hydrogen bonds with a guanine or a modified guanine.

A "deletion," as used herein, refers to a change in either amino acid or nucleotide sequence in which one or more amino acid or nucleotide residues, respectively, are absent.

The term "expressed" or "expression" as used herein refers to the transcription from a gene to give an RNA nucleic acid molecule at least complementary in part to a region of one of the two nucleic acid strands of the gene. The term "expressed" or "expression" as used herein can also refer to the translation of RNA to produce a protein or peptide.

The term "expression vector" as used herein refers to a nucleic acid vector that comprises a gene expression controlling region, such as a promoter or promoter component, operably linked to a nucleotide sequence coding at least one polypeptide.

"Functional portion" or "functional fragment" are used interchangeably and as used herein means a portion or fragment of a whole capable of performing, in whole or in part, a function of the whole. For example, a biologically functional portion of a molecule means a portion of the molecule that performs a biological function of the whole or intact molecule. For example, a functional portion of a gene expression controlling region is a fragment or portion of the specified gene expression controlling region that, in whole or in part, regulates or controls gene expression (*e.g*., facilitates either in whole or in part) in a biological system (*e.g*., a promoter). Functional portions may be of any useful size.

The term "gene expression controlling region" as used herein refers to nucleotide sequences that are associated with a coding sequence and which regulate, in whole or in part, expression of the coding sequence, for example, regulate, in whole or in part, the transcription of the coding sequence. Gene expression controlling regions may be isolated from a naturally occurring source or may be chemically synthesized and can be incorporated into a nucleic acid vector to enable regulated transcription in appropriate cells. The "gene expression controlling regions" may precede, but is not limited to preceding, the region of a nucleic acid sequence that is in the region 5' of the end of a coding sequence that may be transcribed into mRNA.

The terms "heterologous," "exogenous" and "foreign" are used interchangeably herein and in general refer to a biomolecule such as a nucleic acid or a protein that is not normally found in a certain organism or in a certain cell, tissue or other component contained in or produced by an organism. For example, a protein that is heterologous or exogenous to an egg is a protein that is not normally found in the egg. As used herein, the terms "heterologous," "exogenous" and "foreign" with reference to nucleic acids, such as DNA and RNA, are used interchangeably and refer to nucleic acid that does not occur naturally as part of a chromosome, a genome or cell in which it is present or which is found in a location(s) and/or in amounts that differ from the location(s) and/or amounts in which it occurs in nature. It can be nucleic acid that is not endogenous to the genome, chromosome or cell and has been exogenously introduced into the genome, chromosome or cell. Examples of heterologous DNA include, but are not limited to, a DNA comprising a gene expression control region and DNA that encodes a product or products, for example, RNA or protein product. Examples of heterologous DNA include, but are not limited to, gene expression controlling regions or promoters disclosed herein once isolated from the avian and as used thereafter, e.g., after re-introduction into an avian genome.

The term "isolated nucleic acid" as used herein covers, for example, (a) a DNA which has the sequence of part of a naturally occurring genomic molecule but is not flanked by at least one of the sequences that flank that part of the molecule in the genome of the species in which it naturally occurs; (b) a nucleic acid which has been incorporated into a vector or into the genomic DNA of a prokaryote or eukaryote in a manner such that the resulting vector or genomic DNA is not identical to naturally occurring DNA from which the nucleic acid was obtained; (c) a separate molecule such as a cDNA, a genomic fragment, a fragment produced by polymerase chain reaction (PCR), ligase chain reaction (LCR) or chemical synthesis, or a restriction fragment; (d) a recombinant nucleotide sequence that is part of a hybrid gene, i.e., a gene encoding a fusion protein, and (e) a recombinant nucleotide sequence that is part of a hybrid sequence that is not naturally occurring. Isolated nucleic acid molecules of the present disclosure can include, for example, natural allelic variants as well as nucleic acid molecules modified by nucleotide deletions, insertions, inversions, or substitutions.

An "insertion" or "addition," as used herein, refers to a change in an amino acid or nucleotide sequence resulting in the addition of one or more amino acid or nucleotide residues, respectively, as compared to the TAGNPP1 molecule.

The term "nucleic acid" as used herein refers to any linear or sequential array of nucleotides and nucleosides, for example cDNA, genomic DNA, mRNA, tRNA, oligonucleotides, oligonucleosides and derivatives thereof. For ease of discussion, non-naturally occurring nucleic acids may be referred to herein as constructs. Nucleic acids can include bacterial plasmid vectors including expression, cloning, cosmid and transformation vectors such as, animal viral vectors such as, but not limited to, modified adenovirus, influenza virus, polio virus, pox virus, retroviruses such as avian leukosis virus (ALV) retroviral vector, a murine leukemia virus (MLV) retroviral vector, and a lentivirus vector, and the like and fragments thereof. In addition, the nucleic acid can be an LTR of an avian leukosis virus (ALV) retroviral vector, a murine leukemia virus (MLV) retroviral vector, or a lentivirus vector and fragments thereof. Nuclic acids can also include NL vectors such as NLB, NLD and NLA and fragments thereof and synthetic oligonucleotides such as chemically synthesized DNA or RNA. Nucleic acids can include modified or derivatised nucleotides and nucleosides such as, but not limited to, halogenated nucleotides such as, but not only, 5-bromouracil, and derivatised nucleotides such as biotin-labeled nucleotides.

"Nucleic acid sequence" as used herein refers to an oligonucleotide, nucleotide, or polynucleotide, and fragments or portions thereof, and to DNA or RNA of genomic or synthetic origin which may be single- or double-stranded, and represent the sense or antisense strand.

The term "operably linked" refers to an arrangement of elements wherein the components so described are configured so as to perform their usual function. Gene expression controlling regions or promoters (e.g., promoter components) operably linked to a coding sequence are capable of effecting the expression of the coding sequence. The controlling sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

The term "oviduct specific promoter" as used herein refers to promoters and promoter components which are functional, *i*.*e*., provide for transcription of a coding sequence, to a large extent, for example, primarily (*i.e*., more than 50% of the transcription product produced in the animal by a particular promoter type being produced in oviduct cells) or exclusively in oviduct cells of a bird. Examples of useful oviduct specific promoters include, without limitation, ovalbumin promoter, ovomucoid promoter, ovoinhibitor promoter, lysozyme promoter and ovotransferrin promoter and functional portions of these promoters, e.g., promoter components.

The terms "polynucleotide," "oligonucleotide," "nucleotide sequence" and "nucleic acid sequence" can be used interchangeably herein and include, but are not limited to, coding sequences, *i.e*., polynucleotide(s) or nucleic acid sequence(s) which are transcribed and translated into polypeptide in vitro or in vivo when placed under the control of appropriate regulatory or control sequences; controlling sequences, e.g., translational start and stop codons, promoter sequences, ribosome binding sites, polyadenylation signals, transcription factor binding sites, transcription termination sequences, upstream and downstream regulatory domains, enhancers, silencers, DNA sequences to which a transcription factor(s) binds and alters the activity of a gene's promoter either positively (induction) or negatively (repression) and the like. No limitation as to length or to synthetic origin are suggested by the terms described herein.

As used herein, the terms "polypeptide" and "protein" can be used interchangeably and refer to a polymer of amino acids of three or more amino acids in a serial array, linked through peptide bonds. The term "polypeptide" includes proteins such as fusion proteins, protein fragments, protein analogues, oligopeptides and the like. The term "polypeptides" includes polypeptides as defined above that are encoded by nucleic acids, produced through recombinant technology (*e.g*., isolated from a transgenic bird), or chemically synthesized.

As used herein, the term "promoter" refers to a DNA sequence useful to initiate transcription initiation by an RNA polymerase in an avian cell. A "promoter component" is a DNA sequence that can, by itself or, in combination with other DNA sequences effect or facilitate transcription. Specific promoter components such as ovalbumin promoter components, ovomucoid promoter components and lysozyme promoter components and other promoters and promoter components disclosed and claimed herein do not describe a specific promoter sequence. Rather, they encompass any sequence or sequence fragment of the respective promoter that is useful to effect or facilitate transcription of a coding sequence. For example, an ovomucoid promoter component includes, without limitation, the about 1.8 kb, the about 3.9 kb and the about 10 kb ovomucoid promoters disclosed in U.S. Publication No. 11/649,543, published May 17 2007. "Promoter components" can also encompass rearranged gene expression controlling regions which function to initiate RNA transcription and hybrid DNA molecules composed of naturally occurring DNA sequences and/or synthetic DNA sequences which function to initiate RNA transcription.

The terms "recombinant nucleic acid" and "recombinant DNA" as used herein refer to combinations of at least two nucleic acid sequences that are not naturally found in a eukaryotic or prokaryotic cell. The nucleic acid sequences may include, but are not limited to, nucleic acid vectors, gene expression regulatory elements, origins of replication, suitable gene sequences that when expressed confer antibiotic resistance, protein-encoding sequences and the like. The term "recombinant polypeptide" or "recombinant protein" is meant to include a polypeptide produced by recombinant DNA techniques such that it is distinct from a naturally occurring polypeptide either in its location, purity or structure. Generally, such a recombinant polypeptide will be present in a cell in an amount different from that normally observed in nature.

The term "stringent conditions," as used herein, is the "stringency" which occurs within a range from about Tm - 5°C (5 °C below the melting temperature (Tm) of the probe) to about 20°C to 25°C below Tm. As will be understood by those of skill in the art, the stringency of hybridization may be altered in order to identify or detect identical or related polynucleotide sequences.

As used herein, the term "subject" or "patient" encompasses mammals and non-mammals. Examples of mammals include, but are not limited to, humans, chimpanzees, apes monkeys, cattle, horses, sheep, goats, swine; rabbits, dogs, cats, rats, mice, guinea pigs, and the like. Examples of non-mammals include, but are not limited to, birds, fish and the like.

A "substitution," as used herein, refers to the replacement of one or more amino acids or nucleotides by different amino acids or nucleotides, respectively.

As used herein, the term "therapeutically effective amount" refers to any amount of a compound which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein, the terms "TAGNPP1," "fusion protein," "TAGNPP1 polypeptide" and "NPP1 component fused to a targeting moiety" are used interchangeably.

As used herein, the term "treat," "treating" or "treatment" refers to methods of alleviating, abating or ameliorating a disease or condition symptoms, preventing additional symptoms, ameliorating or preventing the underlying causes of symptoms, inhibiting the disease or condition, arresting the development of the disease or condition, relieving the disease or condition, causing regression of the disease or condition, relieving a condition caused by the disease or condition, or stopping the symptoms of the disease or condition either prophylactically and/or therapeutically.

A "variant" of TAGNPP1, as used herein, refers to an amino acid sequence that is altered by one or more amino acids. Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include Asp and Glu; positively charged amino acids include lysine and arginine; and amino acids with uncharged polar head groups having similar hydrophilicity values include Leu, Ile and Val; Gly and Ala; Asp and Gln; and Ser, Thr, Phe and Tyr. Other groups of amino acids that may represent conservative changes include: Ala, Pro, Gly, Glu, Asp, Gln, Asn, Ser, Thr; (2) Cys, Ser, Tyr, Thr; (3) Val, Ile, Leu, Met, Ala, Phe; (4) Lys, Arg, His; and (5) Phe, Tyr, Trp, His. A variant may also, or alternatively, contain nonconservative changes. In a preferred instance, variant polypeptides differ from a native sequence by substitution, deletion or addition of five amino acids or fewer, or by, for example, replacement of a Gly with a Trp. Variants may also (or alternatively) be modified by, for example, the deletion or addition of amino acids that have minimal influence on the immunogenicity, secondary structure and hydropathic nature of the NPP1 component. Guidance in determining which amino acid residues can be substituted, inserted, or deleted without abolishing biological or immunological activity can be found using computer programs well known in the art.

The term "vector" and "nucleic acid vector" as used herein refers to a natural or synthetic single or double stranded plasmid or viral nucleic acid molecule that can be transfected or transformed into cells and replicate independently of, or within, the host cell genome. A circular double stranded vector can be linearized by treatment with an appropriate restriction enzyme based on the nucleotide sequence of the vector. A nucleic acid can be inserted into a vector by cutting the vector with restriction enzymes and ligating the desired pieces together.

The term "portion," as used herein, with regard to a fusion protein refers to fragments of that protein. The fragments may range in size from four amino acid residues to the entire amino acid sequence minus one amino acid. Thus, a protein "comprising at least a portion of the amino acid sequence of SEQ ID NO:1" encompasses the full-length TAGNPP1 and fragments thereof.

"Transformation" or "transfection," as used herein, describes a process by which exogenous DNA enters and changes a recipient cell using various methods well known in the art. Transformation may rely on any known method for the insertion of foreign nucleic acid sequences into a prokaryotic or eukaryotic host cell. The method is selected based on the host cell being transformed and may include, but is not limited to, electroporation, particle bombardment, viral infection, and lipofection. Such "transformed" cells include stably transformed cells in which the inserted DNA is capable of replicating either as an autonomously replicating plasmid or as part of the host chromosome. They also include cells which transiently express the inserted DNA or RNA for limited periods of time.

### EXAMPLES

The present disclosure is further exemplified by the following examples. The examples are for illustrative purpose only and are not intended, nor should they be construed as limiting the invention in any manner.

### Example I (reference)

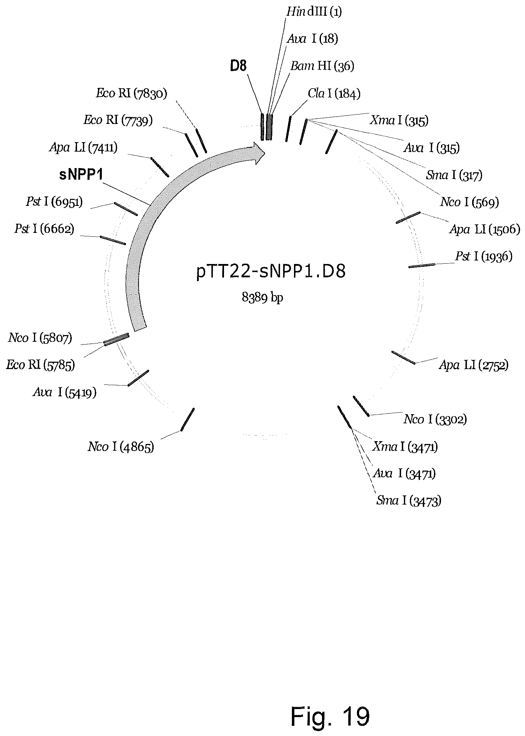

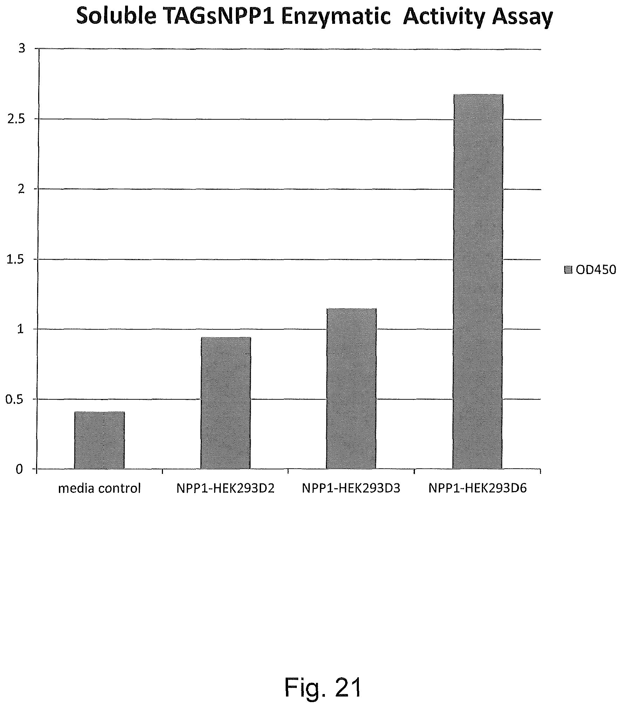

The TAGsNNP1 construct containing the targeting moiety having eight consecutive aspartic acids fused to sNPP1 was ligated into pTT22 vector using EcoRI and HindIII sites (pTT22-sNPP1.D8; Fig. 19). pTT22-sNPP1.D8 was transfected into HEK203E cells and the transformants were cultured to express TAGsNNP1. TAGsNNP1 was isolated from the culture media and partially purified as well known in the art. Following the purification, the pyrophosphase/phosphodiesterase activity of TAGsNPP1 was measured for its ability to hydrolyze thymmidine 5' monophosphate p-nitrophenyl ester. Briefly, TAGsNPP1 was diluted to 1 ng/µL in 50 mM Tris, 250 mM NaCl, pH 9.5. In a plate containing 50 µL of 1 ng/µL TAGsNPP1, 50 µL of 10 mM thymmidine 5' monophosphate p-nitrophenyl ester (Sigma™, Catalog #T4510) substrate was added. The enzyme activity of TAGsNPP1 was measured at 405 nm (absobance) in kinetic mode for 5 minutes. As shown in Fig. 21, the activity of TAGsNPP1 was detected above the level observed in control containing no TAGsNPP1. Particularly, TAGsNPP1 produced from HEK203D6 exhibited the highest level of the enzymatic activity. This results strongly suggested that the truncated NPP1 fused to a targeting moiety (*i.e*., D8) sufficiently maintained its normal function as nuclease.

### Example II (reference)

This non-limiting prophetic example describes how to treat idiopathic infantile arterial calcification by administering a formulation comprising a TAGNPP1 fusion protein.

A clinician uses a diagnostic test to verify that a patient has high levels of calcification in the artery. A genetic test can be also performed for NPP1 defects as described in Rutsch et al. (2003), Nature Genetics 34:379-81.

The pharmaceutical compositions of the present disclosure are preferably administered intravenously, although interadermal, intramuscular or oral administration are employed in certain circumstances.

The clinician determines a dose which may vary depending on the gender, age, health, and weight of the patient. The determination of the appropriate dosage or route of administration is well within the skill of an ordinary physician.

The formulation containing TAGNPP1 can be infused, between about 10 mg/kg and about 1000 mg/kg per week weekly. 10-30 mg/kg can be administered once. During the infusion period, patients are monitored closely and appropriate clinical intervention is taken in the event of an adverse event. Treatment lasts at least 1 month or for the life of the patient. A window of 48 hours may be allowed for each infusion. An infusion schedule in which the rate of infusion increases with time reduces or eliminates adverse events. Infusions for infants can be administered according to the following schedule: 5-10 cc/hr for 60 minutes in each interval.

On the other hand, when continuous intravenous administration is desired, typical example of the slow release systems comprises that 1-100 mg/kg of effective TAGNPP1 proteins can be continuously released for more than 1 day.

### Example III (reference)

### Purification of Soluble TAGsNPP1-D8

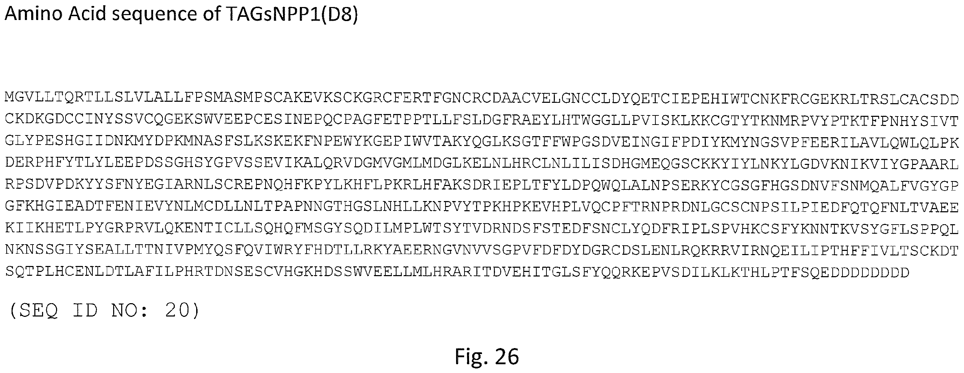

The soluble form of TAGsNPP1 (C-terminally tagged D8) (see, SEQ ID NO:20 and Fig. 26) was purified from HEK293 conditioned medium. The conditioned medium (500ml) was equilibrated to hydroxyapatite (HA)-Buffer A (10mM Na₃PO₄, pH 6.8) and filtered with a 0.2µm Sartobran P Size 8 MidiCap Filter (Sartorius Stedim). A 20 ml HA-Ultrogel column (Pall Life Sciences) was equilibrated with 5 column volume (CV) of Buffer A before loading the filtered conditioned medium at 3ml/min. The column was washed to UV baseline with Buffer A. The protein was eluted stepwise using 2.5CV each of HA-Buffer B (150mM Na₃PO₄, pH 6.8), HA-Buffer C (250mM Na₃PO₄, pH 6.8), and HA-Buffer D (500mM Na₃PO₄, pH 6.8) while collecting 5ml fractions. Active fractions from the HA-column were pooled (50ml) and equilibrated to WGA-Buffer A (20mM Tris, pH 8.0, 150mM NaCl, 0.7% CHAPS). After filtering, 6-7mg total protein (15-18ml) was loaded on a 1ml Wheat Germ Agglutinin (WGA) gravity column (EMD Chemicals) that had been equilibrated with 5CV of WGA-Buffer A. The column was washed with 7CV of WGA-Buffer A, and then the protein was eluted with 5xlml WGA-Buffer B (20mM Tris, pH 8.0, 150mM NaCl, 500mM N-Acetylglucosamine, 0.7% CHAPS buffer). The WGA-Buffer B was allowed to incubate with the column at room temperature for 10 minutes before collecting 1ml fractions. The process was repeated until all of the starting material had been purified by the WGA-column (total of 4 runs). The active fractions (29ml) were pooled and concentrated to 1.2 ml using a 100,000 molecular weight cut off (MWCO) Vivaspin-15 (Sartorius Stedim), buffer exchanging into PBS at the same time.

Active sNPP1-D8 (TAGsNPP1) was purified to 91.5% based on HPLC. When the final pool was analyzed by SDS-PAGE a dimer band corresponded to ∼210kD under non-reducing conditions and under reducing conditions a monomer band corresponded to ∼105kD.

### Example IV (reference)

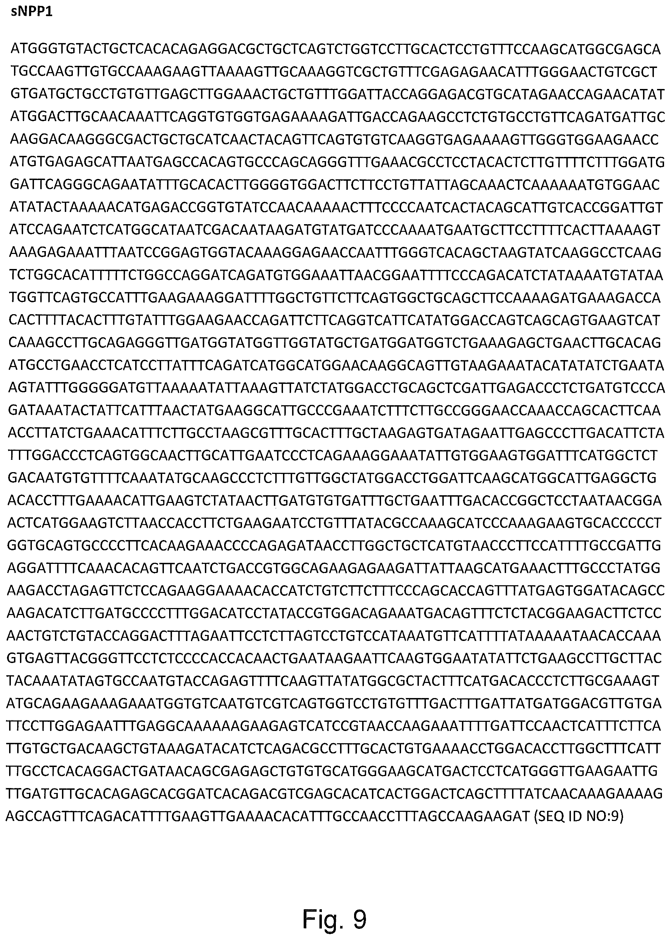

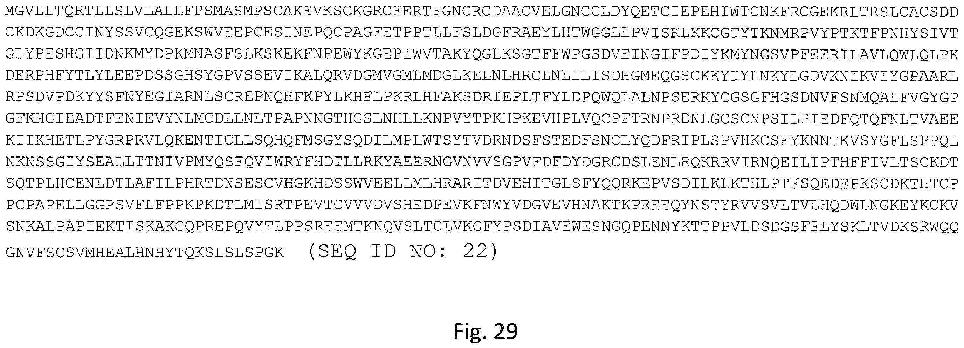

Human aortic smooth muscle cells were plated at 1x10⁴ cells per well in 48 well plates and maintained in Dulbecco's Modified Eagle Medium (DMEM) under standard conditions. After 2 days, DMEM was supplemented with NaPO₄ and ATP at 3.8 mM and 50 uM, respectively. sNPP1 (WT; Fig. 9), TAGsNPP1 having eight consecutive aspartic acid residues (D8) fused to C-terminus (Fig. 26) and sNPP1-Fc (Fig. 29) were supplemented at 1 ug/ml. Media was replaced with same every other day. After 5 days, media was removed from the culture and replaced with 100 ul of 0.6N HCl and incubated for 16-20 hours at room temperature, dissolving calcium phosphates into the solution. Calcium levels were then quantified by a colorimetric Calcium-Cresolphthalein reaction using the Calcium Assay Kit (#700550) from Cayman Chemical Company, Ann Arbor, MI.

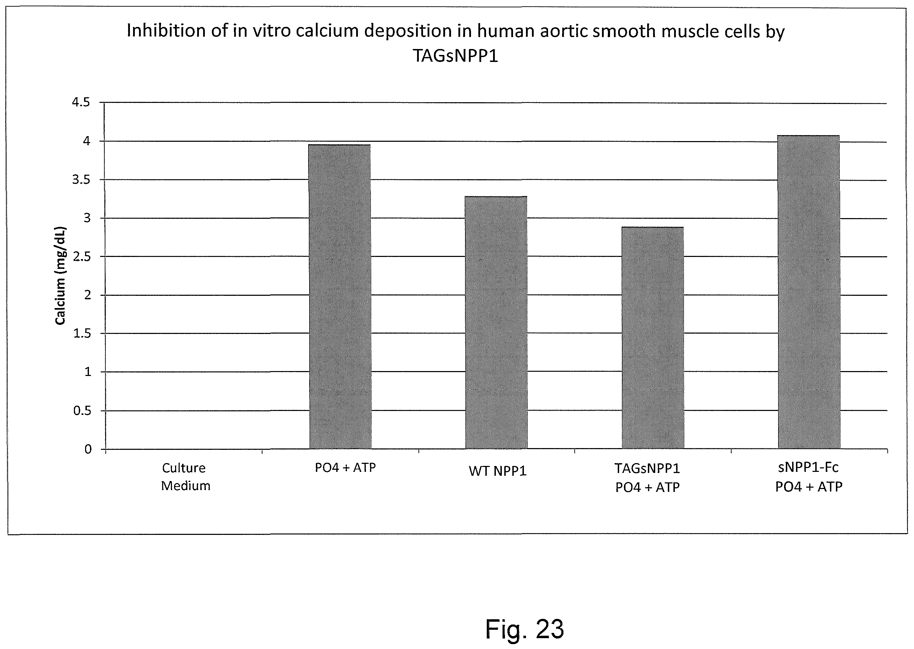

As shown in Fig. 23, TAGsNPP1 demonstrated an increased inhibition on calcification as compared to that of sNPP1 (WT), suggesting that the D8 domain contained in TAGsNPP1 provides an increased homing ability to calcification sites and/or an enhanced inhibitory effect.

### Example V

### Enzymatic Activity Assay of sNPP1-Fc

Addition of 1ug sNPP1-Fc (Fig. 29) was based on 70% purity (*i.e.* ∼1.1µg of sNPP1-Fc was used in the assay). HPLC size exclusion column (SEC) indicates that the sNPP1-Fc is ∼78% pure. The non-reduced gel showed a dimer at the expected size (∼250kD). The dimer band reduced to the expected monomer size (∼125kD) when treated with DTT. Western blot analysis confirmed the dimer and monomer bands. Treatment of the sNPP1-Fc sample with PNGase F reduced the monomer band from ∼125kD to ∼100kD, which is the expected molecular weight considering that sNPP1-Fc contained 12 N-glycan sites. At 78% purity, final quantitation yielded ∼0.808mg sNPP1-Fc (2.2µg/ml conditioned media). The enzymatic activity of sNPP1-Fc was determined as known in the art.

**Table 1. Enzymatic activity of sNPP1-Fc**

| **Sample** | **OD₄₀₅** | **Final OD₄₀₅** |
|---|---|---|
| Neg. Cntrl (non-NPP1 protein) | 0.053 | 0.000 |
| 1ug Wt sNPP1 (soluble sNPP1) | 0.296 | 0.243 |
| 1ug NPP1-Fc (c-terminus) | 0.397 | 0.344 |

Each example in the above specification is provided by way of explanation of the disclosure, not limitation. In fact, it will be apparent to those skilled in the art that various modifications, combinations, additions, deletions and variations can be made in the present invention without departing from the scope of the invention encompassed by the appended claims. For instance, features illustrated or described as part of one embodiment can be used in another embodiment to yield a still further embodiment.

## Claims

1. An isolated polypeptide consisting essentially of an NPP1 component and an Fc region of an immunoglobulin, wherein the NPP1 component is a truncated NPP1 comprising the cysteine rich region and a catalytic domain having pyrophosphatase and/or phosphodiesterase activity but having the N-terminal cytosolic and transmembrane domains removed, and wherein said Fc region is linked to the C-terminus of the NPP1 component.

2. The polypeptide of Claim 1, wherein said NPP1 component has the amino acid sequence set forth in P99 through D925 of SEQ ID NO: 1.

3. The polypeptide of Claim 1 having the amino acid sequence set forth in SEQ ID NO: 22, optionally without the signal peptide at at its N-terminus.

4. The polypeptide of Claim 3, wherein said polypeptide forms a homodimer.

5. An isolated nucleic acid encoding the polypeptide of Claim 1, wherein the nucleic acid comprises a DNA sequence encoding the NPP1 component and a DNA sequence encoding the Fc region, wherein the 3' end of the DNA sequence encoding the NPP1 component is ligated, with or without a linker, to the 5' end of the DNA sequence encoding the Fc region so that the reading frames of the sequences are in phase.

6. The nucleic acid of claim 5 having a nucleotide sequence as set forth in SEQ ID NO: 21.

7. An expression vector carrying the isolated nucleic acid of Claim 5.

8. A process for producing an isolated polypeptide as claimed in claim 1, the process comprising expression of the DNA sequences of claim 5 into a single fusion protein that has the biological activity of said NPP1 component and said Fc region.

9. A pharmaceutical composition comprising the polypeptide of any one of Claims 1 to 3 in combination with a pharmaceutically acceptable carrier, diluent or excipient.

10. A pharmaceutical composition comprising the isolated polypeptide of Claim 1 for use in therapy.

11. A pharmaceutical composition comprising the isolated polypeptide of Claim 1 for use in the treatment of arterial blockage, generalized arterial calcification of infancy, insulin resistance, hypophosphatemic rickets, or ossification of the posterior longitudinal ligament of the spine.

## Patentansprüche

1. Isoliertes Polypeptid bestehend im Wesentlichen aus einer NPP1-Komponente und einer Fc-Region von einem Immunglobulin, wobei die NPP1-Komponente ein trunkiertes NPP1 ist, umfassend die cysteinreiche Region und eine katalytische Domäne mit Pyrophosphatase- und/oder Phosphodiesterase-Aktivität, aber mit den N-terminalen cytosolischen und transmembranen Domänen entfernt, und wobei die genannte Fc-Region mit dem C-Terminus der NPP1-Komponente verknüpft ist.

2. Polypeptid nach Anspruch 1, wobei die genannte NPP1-Komponente die in P99 bis D925 von SEQ ID NO: 1 dargelegte Aminosäuresequenz aufweist.

3. Polypeptid nach Anspruch 1, das die in SEQ ID NO: 22 dargelegte Aminosäuresequenz aufweist, gegebenenfalls ohne das Signalpeptid an seinem N-Terminus.

4. Polypeptid nach Anspruch 3, wobei das genannte Polypeptid ein Homodimer bildet.

5. Isolierte Nukleinsäure, kodierend für das Polypeptid nach Anspruch 1, wobei die Nukleinsäure eine für die NPP1-Komponente kodierende DNA-Sequenz und eine für die Fc-Region kodierende DNA-Sequenz umfasst, wobei das 3'-Ende der DNA-Sequenz, die für die NPP1-Komponente kodiert, mit einem oder ohne einen Linker, an das 5'-Ende der DNA-Sequenz, die für die Fc-Region kodiert, dergestalt ligiert ist, dass die Leserahmen der Sequenzen in Phase sind.

6. Nukleinsäure nach Anspruch 5 mit einer Nukleotidsequenz wie in SEQ ID NO: 21 dargelegt.

7. Expressionsvektor, der die isolierte Nukleinsäure nach Anspruch 5 trägt.

8. Verfahren zum Produzieren eines isolierten Polypeptids nach Anspruch 1, wobei das Verfahren die Expression der DNA-Sequenzen nach Anspruch 5 in ein einzelnes Fusionsprotein umfasst, das die biologische Aktivität der genannten NPP1-Komponente und der genannten Fc-Region aufweist.

9. Pharmazeutische Zusammensetzung, umfassend das Polypeptid nach einem der Ansprüche 1 bis 3 in Kombination mit einem pharmazeutisch verträglichen Träger, Verdünnungsmittel oder Hilfsstoff.

10. Pharmazeutische Zusammensetzung, umfassend das isolierte Polypeptid nach Anspruch 1, zur Verwendung in der Therapie.

11. Pharmazeutische Zusammensetzung, umfassend das isolierte Polypeptid nach Anspruch 1 zur Verwendung bei der Behandlung eines Arterienverschlusses, einer generalisierten infantilen Arterienkalzifikation, einer Insulinresistenz, einer hypophosphatämischen Rachitis oder einer Ossifikation des hinteren Längsbandes der Wirbelsäule.

## Revendications

1. Polypeptide isolé consistant essentiellement en un constituant NPP1 et en une région Fc d'une immunoglobuline, dans lequel le constituant NPP1 est une protéine NPP1 tronquée comprenant la région riche en cystéine et un domaine catalytique présentant une activité pyrophosphatase et/ou phosphodiestérase, mais dont les domaines cytosoliques et transmembranaires N-terminaux ont été retirés, et dans lequel ladite région Fc est liée à la terminaison C du constituant NPP1.

2. Polypeptide selon la revendication 1, dans lequel ledit constituant NPP1 a la séquence d'acides aminés énoncée dans P99 à D925 de la SÉQ ID n° 1.

3. Polypeptide selon la revendication 1, ayant la séquence d'acides aminés énoncée dans la SÉQ ID n° 22, optionnellement sans le peptide signal à sa terminaison N.

4. Polypeptide selon la revendication 3, ledit polypeptide formant un homodimère.

5. Acide nucléique isolé codant le polypeptide selon la revendication 1, l'acide nucléique comprenant une séquence d'ADN codant le constituant NPP1 et une séquence d'ADN codant la région Fc, dans lequel l'extrémité 3' de la séquence d'ADN codant le constituant NPP1 est ligaturée, avec ou sans lieur, à l'extrémité 5' de la séquence d'ADN codant la région Fc de telle sorte que les cadres de lecture des séquences soient en phase.

6. Acide nucléique selon la revendication 5, ayant une séquence nucléotidique telle qu'énoncée dans la SÉQ ID n° 21.

7. Vecteur d'expression portant l'acide nucléique isolé selon la revendication 5.

8. Procédé de production d'un polypeptide isolé selon la revendication 1, le procédé comprenant l'expression des séquences d'ADN selon la revendication 5 dans une seule protéine de fusion qui a l'activité biologique dudit constituant NPP1 et de ladite région Fc.

9. Composition pharmaceutique comprenant le polypeptide selon l'une quelconque des revendications 1 à 3 en combinaison avec un support, un diluant ou un excipient pharmaceutiquement acceptable.

10. Composition pharmaceutique comprenant le polypeptide isolé selon la revendication 1, destinée à une utilisation thérapeutique.

11. Composition pharmaceutique comprenant le polypeptide isolé selon la revendication 1, destinée à une utilisation dans le traitement d'un blocage artériel, d'une calcification artérielle généralisée infantile, d'une insulinorésistance, d'un rachitisme hypophosphatémique ou d'une ossification du ligament longitudinal postérieur de la colonne vertébrale.
